# EUROPEAN PATENT APPLICATION

(11) **EP 4 140 395 A2**
(43) Date of publication of application: **01.03.2023**
(21) Application number: 22179179.1
(22) Date of filing: 15.06.2022
(51) Int. Cl.: A61B 5/00, A61B 5/08, A61H 31/00, A61M 11/00, A61M 16/00, A61M 16/04, A61M 16/06, A61M 16/10, A61M 16/14, A61M 16/20, A61N 1/39, G01F 1/00, G16H 20/40

(54) **SYSTEMS AND METHODS FOR AIRWAY MANAGEMENT**

(30) Priority: 15.06.2021 US 202163210890 P
(71) Applicant: Stryker Corporation, Kalamazoo, MI 49002 (US)
(72) Inventor: CHAPMAN, Fred W., Kalamazoo, 49002 (US); WALKER, Robert G., Kalamazoo, 49002 (US)
(74) Representative: V.O.

(57) **Abstract**

Monitors for evaluating airway procedures, particularly in a pre-hospital environment, are described herein. In an example method, an airway parameter of an individual receiving assisted ventilation is detected by an airway sensor. A monitor determines a metric based on the airway sensor. Further, the monitor performs an action based on the metric.

## Description

### CROSS-REFERENCE TO RELATED APPLICATION

This application claims the priority of U.S. Provisional Application No. 63/210,890, which was filed on June 15, 2021 and is incorporated by reference herein in its entirety.

### BACKGROUND

In some emergency medical care circumstances, procedures and devices are utilized to establish or ensure safe and effective passage of air into and out of a patient's lungs. The application of such procedures and devices is commonly referred to as airway management. In one of several types of airway management, a patient is administered positive pressure ventilation (PPV). Ordinary breathing by the patient is a negative pressure phenomenon - the diaphragm and chest wall move to increase the volume of the pleural cavity, reducing the pressure in the lungs to below atmospheric pressure, which pulls air into the lungs. When patients are receiving PPV, a positive pressure is created in a device (e.g. a mechanical ventilator or a manual resuscitation bag) attached to the patient's airway, forcing air into the lungs. PPV inverts and/or alters much of the physiology of normal breathing, which leads to many different challenges and hazards associated with delivery of PPV.

### SUMMARY OF THE INVENTION

According to an aspect is provided a medical device, comprising a sensor configured to detect an airway parameter of an individual receiving positive pressure ventilation (PPV) from a ventilation device, an output device, and a processor. The airway parameter can comprise one or more of a pressure of air in the airway, a flow rate of the air in the airway, a volume of the air in the airway, a rate of respiration and/or ventilation, an inspiratory time, an expiratory time, a partial pressure of CO₂ in the airway, a partial pressure of O₂ in the airway, an end tidal CO₂, and an end tidal O₂. The processor is configured to determine that a leak is present between the ventilation device and an airway of the individual by analyzing the airway parameter. The processor is configured to, in response to determining that the leak is present between the ventilation device and the airway of the individual, cause the output device to output an alert indicating the leak.

Optionally, the the airway parameter comprises a partial pressure of CO₂ in the airway of the individual, and the processor is configured to determine that the leak is present between the ventilation device and the airway of the individual by determining that the airway parameter lacks a plateau phase. Alternatively, or additionally, the airway parameter comprises a partial pressure of O₂ in the airway of the individual, and the processor is configured to determine that the leak is present between the ventilation device and the airway of the individual by determining that the airway parameter is below a threshold. Alternatively, or additionally, the airway parameter comprises a volume of air in the airway of the individual, and the processor is configured to determine that the leak is present between the ventilation device and the airway of the individual by determining that the airway parameter is lower than a volume of air output by the ventilation device.

Optionally, the processor is configured to determine that the leak is present between the ventilation device and the airway of the individual by analyzing first data indicating the airway parameter detected by the sensor during a first time interval. The processor can then be further configured to determine that a medication has prevented the individual from breathing by analyzing second data indicating the airway parameter detected by the sensor during a second time interval, and based on determining that the medication has prevented the individual from spontaneously breathing, cause the output device to output a recommendation to connect the ventilation device to the airway of the individual. Alternatively, or additionally, the processor can then be further configured to identify breath events by analyzing second data indicating the airway parameter detected by the sensor during a second time interval, the breath events comprising a positive pressure ventilation (PPV) breath event. Herein, the processor can be configured to identify a spontaneous breath event among the breath events, and in response to identifying the spontaneous breath event, outputting an indication of the spontaneous breath event.

Optionally, the medical device further comprises a blood pressure sensor configured to detect a blood pressure of the individual. Optionally, the airway parameter comprises an end tidal CO₂, and the processor is further configured to determine a time interval during which the airway parameter is less than a first threshold and a respiration rate of the individual is greater than a second threshold. The processor can be configured to determine that the individual is being hyperventilated by determining a hyperventilation index using the time interval, and in response to determining that the individual is being hyperventilated, cause the blood pressure sensor to detect the blood pressure of the individual.

According to an aspect is provided a system comprising a ventilation device configured to be connected to an airway of a patient and a medical device, e.g. as described herein above. The medical device can comprise a sensor configured to detect an airway parameter of an individual receiving positive pressure ventilation (PPV) from a ventilation device, an output device, and a processor.

Optionally, the sensor is a CO₂ sensor configured to detect a partial pressure of CO₂ in the airway of the patient.

Optionally, the output device is a display configured to output a first waveform indicating the partial pressure of CO₂ during a time interval and a second waveform indicating an airway parameter that is different than the partial pressure of CO₂ during the time interval. The time interval can be a first time interval.

Optionally, the processor is configured to identify a breath event in the partial pressure of CO₂ of the airway during the time interval. The processor can be configured to identify a leak between the ventilation device and the airway by determining that the breath event lacks a plateau phase. The processor can be configured to determine that the breath event lacks a plateau phase by determining that a slope of the breath event following the maximum partial pressure of CO₂ in the breath event is greater than a first threshold, and/or determining that a time interval between a maximum partial pressure of CO₂ and a local minimum partial pressure of CO₂ after the maximum partial pressure of CO₂ is greater than a second threshold. The processor can be configured to, in response to determining that the breath event lacks the plateau phase, cause the output device to output a first alert warning to check for the leak between the ventilation device and the airway of the patient. The processor can be configured to cause the output device to output a second alert indicating that the airway parameter indicated by the second waveform is erroneous.

According to an aspect is provided a system comprising a ventilation device configured to be connected to an airway of a patient, a CO₂ sensor configured to detect a partial pressure of CO₂ in the airway of the patient, a display configured to output a first waveform indicating the partial pressure of CO₂ during a time interval and a second waveform indicating an airway parameter that is different than the partial pressure of CO₂ during the time interval, and a processor. The time interval can be a first time interval. The processor is configured to identify a breath event in the partial pressure of CO₂ of the airway during the time interval, and to identify a leak between the ventilation device and the airway by determining that the breath event lacks a plateau phase. The processor can be configured to determine that the breath event lacks a plateau phase by determining that a slope of the breath event following the maximum partial pressure of CO₂ in the breath event is greater than a first threshold, and/or determining that a time interval between a maximum partial pressure of CO₂ and a local minimum partial pressure of CO₂ after the maximum partial pressure of CO₂ is greater than a second threshold. The processor can be configured to, in response to determining that the breath event lacks the plateau phase, cause the display to output a first alert warning to check for the leak between the ventilation device and the airway of the patient. The processor can be configured to cause the display to output a second alert indicating that the airway parameter indicated by the second waveform is erroneous.

Optionally, the processor is further configured to determine that a paralytic has prevented the individual from breathing by analyzing first data, the first data indicating the airway parameter during a second time interval. The processor can be configured to, based on determining that the paralytic has prevented the individual from spontaneously breathing, cause the display to output a recommendation to connect the ventilation device to the airway of the individual. The processor can be configured to identify breath events by analyzing second data, the second data indicating the airway parameter during a third time interval, the breath events comprising a positive pressure ventilation (PPV) breath event. The processor can be configured to identify a spontaneous breath among the breath events. The processor can be configured to, in response to identifying the spontaneous breath event, cause the display to output an indication of the spontaneous breath event.

Optionally, the system further comprises a blood pressure sensor configured to detect a blood pressure of the individual. The processor can be further configured to determine a second time interval during which the partial pressure of CO₂ is less than a first threshold and a respiration rate of the individual is greater than a second threshold. The processor can be configured to determine that the individual is being hyperventilated by determining a hyperventilation index proportional to a duration of the second time interval. The processor can be configured to, in response to determining that the individual is being hyperventilated, cause the blood pressure sensor to detect the blood pressure of the individual.

According to an aspect is provided a method, comprising identifying data indicating an airway parameter of an individual detected during a time interval, the data being indicative of breath events comprising a positive pressure ventilation (PPV) breath event. The method comprises identifying a spontaneous breath event among the breath events; and in response to identifying the spontaneous breath event, outputting an indication of the spontaneous breath event.

The time interval can be a first time interval. The data can be first data. The airway parameter can be a first airway parameter. The spontaneous breath event can be a first spontaneous breath event,

Optionally, the airway parameter comprises a pressure of air in an airway of the individual, a flow rate of the air in the airway, a volume of the air in the airway, a rate of respiration and/or ventilation, an inspiratory time, an expiratory time, a partial pressure of CO₂ in the airway, a partial pressure of O₂ in the airway, an end tidal CO₂, or an end tidal O₂.

Optionally, the method further comprises identifying second data indicating the airway parameter of the individual detected during a second time interval. The method can comprise determining that a medication has prevented the individual from breathing by determining that a portion of the second data lacks a second spontaneous breath event during the second time interval. The method can comprise, in response to determining that the medication has prevented the individual from spontaneously breathing, outputting a recommendation to connect a ventilation device to an airway of the individual.

Optionally, identifying the spontaneous breath event among the breath events comprises determining a spontaneous breathing index by analyzing the data, the spontaneous breathing index indicating a contribution of spontaneous breaths in breathing of the individual during the time interval, and comparing the spontaneous breathing index to a threshold.

Alternatively, or additionally, identifying the spontaneous breath event among the breath events comprises determining a variability index by analyzing the data, the variability index indicating a variance of the airway parameter during the time interval, and comparing the variability index to a threshold.

Optionally, the method further comprises detecting a second airway parameter of the individual during the time. The method can comprise generating a corrected airway parameter by decreasing the second airway parameter. The method can comprise outputting the corrected airway parameter.

Optionally, outputting the indication of the spontaneous breath event comprises outputting an alert indicating that the individual is gaining spontaneous respiration, outputting an instruction to administer a medication to the individual, and/or transmitting data indicating the spontaneous breath event to an external device.

Optionally, the method further comprises identifying an input signal indicating that a ventilation device was placed during a second time interval. The method cn comprise identifying second data indicating the airway parameter of the individual detected during the second time interval. The method can comprise determining that the second data indicates an absence of breath events during the second time interval. The method can comprise in response to determining that the second data indicates the absence of the breath events during the second time interval, outputting an alert indicating that the input signal is erroneous.

Optionally, the method further comprises identifying second data indicating the airway parameter of the individual detected during a second time interval. The method can comprise determining that a leak is present between the ventilation device and the airway of the individual by analyzing the second data. The method can comprise, in response to determining that the leak is present between the ventilation device and the airway of the individual ouputtting an alert indicating the leak.

It will be appreciated that any of the aspects, features and options described herein can be combined. It will particularly be appreciated that any of the aspects, features and options described in view of the medical device apply equally to the system and the method, and vice versa.

### BRIEF DESCRIPTION OF THE DRAWINGS

FIG. 1 illustrates an example rescue scene that includes a rescuer managing care of a patient.
FIG. 2 illustrates an example patient receiving assisted ventilation via a mask.
FIG. 3 illustrates an example intubated patient receiving assisted ventilation via an endotracheal tube.
FIG. 4 illustrates an example of a bidirectional airflow sensor.
FIG. 5 illustrates an example process for analyzing the airway of an individual.
FIG. 6 illustrates an example process for evaluating spontaneous breathing during PPV of a patient.
FIG. 7 illustrates an example process for evaluating the accuracy of an airway parameter.
FIG. 8 illustrates an example of an external defibrillator configured to perform various functions described herein.

### DETAILED DESCRIPTION

Several different forms of PPV may be utilized in an emergency medical care situation. Examples include non-invasive PVV. In some cases, the patient receives PPV delivered via a bag-valve-mask (BVM) device. With a BVM device, a facemask is placed over the patient's mouth and nose, and a medical care provider squeezes a self-inflating bag attached to the facemask to deliver PPV. While BVM ventilation is a very common method of PPV, performed in emergency medical care situations by all levels of medical care providers, it poses several challenges and hazards. For example, the mask may not form an adequate seal with the patient's face, resulting in an air leak that may compromise ventilation effectiveness and also compromise the accurate measurement of some ventilation parameters. For another example, the self-inflating bag typically holds a volume of air much larger than what should optimally be delivered to the patient, creating a possibility for excessive and/or inconsistent ventilation volumes. Additionally, the bag can be squeezed in a manner that can create excessive airway pressures, which can result in gastric insufflation and regurgitation of stomach contents. This can then complicate further airway management by contaminating the airway, and can cause direct patient harm if the regurgitated stomach contents are aspirated into the lungs. There is thus a need and opportunity to assist medical care providers with optimal use of a BVM device and optimal delivery of ventilation via a BVM.

In some cases, the patient receives PPV delivered through a device inserted into the airway. Examples of such devices include an endotracheal tube (ETT), and a supraglottic airway (SGA). Placement of such invasive airway devices is sometimes accompanied by administration of medications to better facilitate prompt and successful airway placement. Once the airway is inserted, PPV is frequently delivered manually by squeezing a self-inflating bag attached to the airway; consequently there can be challenges and hazards related not only to proper placement and sealing of the airway, but also related to ventilation volumes, pressures, and overall ventilation and oxygenation effectiveness.

One particular type of airway management involving placement of an invasive airway after administration of certain medications is commonly known as rapid sequence intubation (RSI, also referred to as rapid sequence induction). This is a category of advanced airway management procedures performed on patients not in cardiac arrest, where medications are used to rapidly create optimal intubation conditions, maximizing the likelihood of prompt success in placing an artificial airway (e.g. an ETT or an SGA). In RSI, before the artificial airway is placed, the patient is administered anesthesia in a process known as "induction." The anesthesia administered to the patient includes medications to induce temporary sedation and paralysis (also referred to as neuromuscular blockade or NMB); sometimes additional medications are included as well.

Several variants of RSI are also possible, involving variations in details such as the relative timing of administration of the different medications, or the type of airway device that is placed. Examples of some terms used for some of these RSI variants include rapid sequence airway (RSA), wherein the intended artificial airway device may be a supraglottic airway rather than an endotracheal tube, and delayed sequence intubation (DSI), wherein sedation is induced several minutes before administration of the paralytic medication to allow more effective pre-oxygenation of patient, which can improve patient safety during the ensuing period of paralytic-induced apnea while the care provider attempts to place the airway device.

RSI and its variants can be some of the most complex, stressful, and risky procedures performed on patients by emergency medical service providers. Emergency RSI attempts can result in rapid physiologic deterioration of the patient, sometimes leading to cardiac arrest. The process of RSI impacts many different aspects of the patient's physiology, which can lead to a variety of harms. Thus, there is a huge need and opportunity for improvement of RSI procedures, which can directly translate into saving lives.

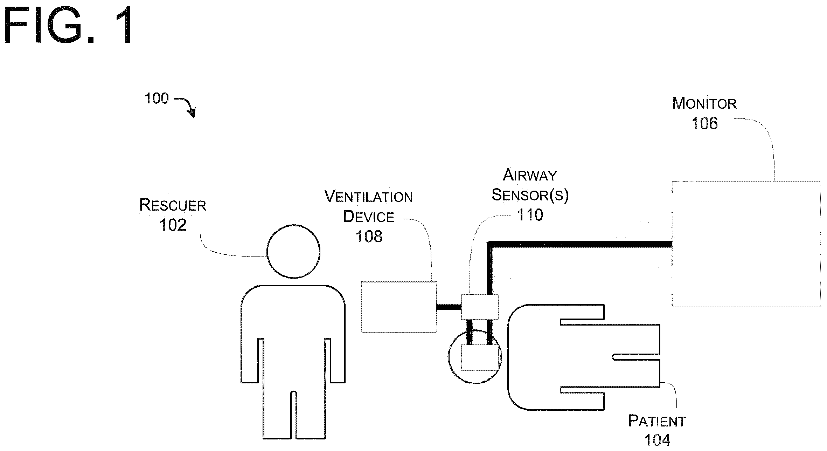

FIG. 1 illustrates an example rescue scene 100 that includes a rescuer 102 managing care of a patient 104. In particular, the rescuer 102 utilizes a monitor 106 to elucidate one or more parameters of the patient 104. In various cases, a ventilation device 108 provides assisted ventilation to the patient 104 at the rescue scene 100. In various examples, the rescue scene 100 is outside of a formal clinical environment. For example, the rescue scene 100 may occur outdoors, in a home, in a workplace, in a patient transport environment (e.g., an ambulance, helicopter, etc.), or the like. In some cases, the rescue scene 100 is a pre-hospital environment, such that the patient 104 is actively being transported or transferred to a hospital environment. Accordingly, the monitor 106 and/or the ventilation device 108 may be portable devices capable of monitoring and/or treating the patient 104 outside of a clinical environment.

In various implementations, the monitor 106 includes at least one processor configured to perform one or more operations associated with tracking a physiological condition of the patient 104. In some cases, the monitor 106 further includes memory configured to store instructions that are executed by the processor(s). In some cases, the monitor 106 is a medical device. The monitor 106 is configured to track a physiological condition of the patient 104. In some cases, the monitor 106 is also configured to administer a therapy to the patient 104. For instance, the monitor 106 is a monitor-defibrillator that is also configured to administer an electrical shock to the patient. According to some cases, the monitor 106 is a computing device that includes a processor configured to perform various operations based on the physiological condition of the patient 104. In some examples, the monitor 106 is a tablet computer, a personal computer, a mobile phone, or another type of user equipment. In various instances, the monitor 106 is a wearable device, such as a smart watch or smart glasses. For example, the monitor 106 is worn by the rescuer 102 at the rescue scene 100.

The monitor 106 includes and/or receives data from one or more sensors. The data is indicative of one or more parameters of the patient 104. In various cases, the parameter(s) include at least one of an electrocardiogram (ECG) of the patient 104, a temperature of the patient 104, a blood pressure of the patient 104, a blood oxygenation (e.g., a regional oxygenation (rSO₂) or an arterial oxygen saturation such as determined by a pulse-oximeter (spO₂)) of the patient 104, a heart rate of the patient 104, a pulse rate of the patient 104, or one or more airway parameters. As used herein, the term "airway parameter," and its equivalents, may refer to a metric related to and/or indicative of air present in and/or flowing through lungs, a trachea, a throat, or mouth of a patient. Examples of airway parameters include parameters indicative of a characteristic of the airway of the patient 104, or a characteristic of the respirations of the patient 104, or a characteristic of ventilations being provided to the patient 104, such as airway pressure (e.g., positive end-expiratory pressure (PEEP), plateau pressure, peak inspiratory pressure (PIP), etc.), airway flow, airway volume (e.g., inspiratory volume, expiratory volume, tidal volume, or minute volume), respiration/ventilation rate, inspiratory and/or expiratory time, partial pressure of CO₂, end tidal CO₂, partial pressure of O₂, end tidal O₂, and so on.

In various cases, the ventilation device 108 is a medical device. According to various implementations, the ventilation device 108 includes a gas source, an element configured to move the gas in and out of the airway of the patient 104, as well as an interface configured to connect the ventilation device 108 to the airway of the patient 104. For instance, the gas source may include an O₂ tank or other container for an O₂-containing gas that is administered to the patient 104. The element configured to move the gas in and out of the airway of the patient 104, in some cases, includes a manual device such as a bag-valve device (e.g., a bag that is manually squeezed by the rescuer 102 in order to propel the gas into the airway of the patient 104 and that is manually released by the rescuer in order to allow exhalation from the patient 104) or an automated device such as a mechanical ventilator. In some implementations, the interface includes a mask that is disposed on the face of the patient 104 (e.g., over the mouth and nose of the patient 104), an endotracheal tube disposed in the trachea of the patient 104, or a supraglottic device disposed in a pharynx of the patient 104. The ventilation device 108, in various cases, includes at least one duct (e.g., a tube, pipe, or the like) that is fluidly connected to the airway of the patient 104, and through which air is moved into, through, and out of the airway of the patient 104. In some cases, the ventilation device 108 includes a bag-valve mask that includes a number of valves, including a valve configured to prevent exhaled air from flowing back into the bag, thus avoiding rebreathing of expired CO₂. A supraglottic device, in various examples, includes a bag attached to a duct that fits over or otherwise directs air to the glottic opening of the patient 104, without being inserted through the vocal cords. In various examples, the ventilation device 108 includes a valve that selectively vents a fluid circuit connecting an interior of the ventilation device 108 and the airway of the patient 104 to an external environment.

In some instances, the ventilation device 108 is communicatively coupled to the monitor 106 via one or more wired interfaces, one or more wireless interfaces, or a combination thereof. For example, the ventilation device 108 may include a processor configured to cause assisted ventilation to be administered to the patient 104, and the monitor 106 may transmit a signal to the ventilation device 108 that causes the processor to adjust a parameter of the assisted ventilation (e.g., a ventilation rate, a length of a ventilation pause, a volume of air administered to the patient 104, a flow rate of air administered to the patient 104, or any combination thereof) and/or activates the valve in order to vent the ventilation device 108.

In various cases, the airway parameter(s) are detected by one or more airway sensors 110. In some instances, the airway sensor(s) 110 are included in, or integrated with, the monitor 106, the ventilation device 108, or a combination thereof. For example, the airway sensor(s) 110 are integrated with and/or attached to a mechanical ventilator, a bag-valve mask, an endotracheal tube, a duct of the ventilation device 108, airway adaptors, or any other device fluidly connected to the airway of the patient 104. According to some cases, the airway sensor(s) 110 are communicatively coupled to a processor of the monitor 106 and/or a processor of the ventilation device 108. For example, the airway sensor(s) 110 are connected via one or more wired interfaces, one or more wireless interfaces, or a combination thereof.

The airway sensor(s) 110 include one or more types of sensors configured to detect the airway parameter(s). In various cases, the airway sensor(s) 110 include an electrical circuit configured to generate an analog signal (e.g., an electrical current or voltage) based on the airway parameter(s) of the patient 104. Optionally, the airway sensor(s) 110 include an analog-to-digital converter (ADC) that converts the analog signal into a digital signal. In particular implementations, the airway sensor(s) 110 include a transmitter and/or transceiver configured to transmit a signal to the monitor 106 and/or the ventilation device 108 based on the digital signal. In some examples, the airway sensor(s) 110 include a pressure-gradient flowmeter, an ultrasound-based flow sensor, a turbine-based flowmeter, a wire anemometer (including, e.g., a heated wire that changes temperature and resistance based on the magnitude of airflow it is exposed to), an analogic differential pressure flow sensor, or a combination thereof.

A pressure-gradient flowmeter, for example, operates by inducing a pressure differential in the duct. In some examples, the pressure-gradient flow meter includes an element that induces the pressure differential on either side of the element. The pressure-gradient flow meter, in various implementations, includes a pressure sensor configured to detect the difference between the sides of the element. For instance, the pressure-gradient flowmeter includes a diaphragm exposed to pressure from one side of a resistor on one of its sides, and exposed to pressure from the other side of the resistor on its other side. The diaphragm includes and/or is integrated with strain gauges, for example. Thus, in various cases, the pressure-gradient flowmeter directly measures the difference in pressures and is configured to measure bi-directional flow through the duct. The flow rate through the sensor is determined based on the different pressures, in accordance with Bernoulli's principle. In some implementations, a high-efficiency particulate absorbing filter (HEPA) filter induces the pressure differential in the pressure-gradient flowmeter. In addition to inducing the pressure differential, the HEPA filter prevents the passage of airborne diseases from the patient 104 through the duct, which can protect the rescuer 102.

In some implementations, the airway sensor(s) 110 includes a bidirectional airflow sensor, which is configured to detect both inspiratory and expiratory airflow through the airway of the patient 106. For instance, the airway sensor(s) 110 includes a two-way thermal mass sensor, such as the sensor described in U.S. Pub. No. 2018/0160970, which is incorporated herein by reference. The two-way thermal mass sensor measures a flow rate of air through a bidirectional duct in both directions. When the duct is fluidly connected to the airway of the patient 104, the flow rate may be indicative of the flow rate of air entering the lungs of the patient 106 on insufflation and the flow rate of air expired from the lungs of the patient 106 on expiration. The two-way thermal mass sensor, for instance, detects a flow rate based on a temperature gradient that is correlated to the amount of air flowing through the duct. Unlike some pressure-gradient flowmeters, the two-way thermal mass sensor provides negligible resistance to air flow.

In various examples, the two-way thermal mass sensor includes a heater element mounted in the interior of the duct, as well as at least one temperature sensor upstream from the heater element in the interior of the air duct, and at least one temperature sensor downstream from the heater element in the interior of the air duct. Air flow through the air duct causes a transfer of heat from the heater element to the air, which is detectable by at least one of the temperature sensors. For example, the two-way thermal mass sensor has a sensor dead space < 10 mL and airway resistance < 1.8 cmH2).L.S.s-1 at 60 L.min-1).

In some cases, the airway sensor(s) 110 is connected to memory and/or at least one processor that generates digital data indicative of the flow rate detected by the airway sensor(s) 110. Based on the flow rate, the processor(s) may be configured to generate data indicative of inspiratory/expiratory volume, tidal volume, ventilation rate, inspiratory/expiratory time, amount of leakage of the patient 106 at each ventilation cycle. In some cases, the airway sensor(s) 110 is single-use and/or autoclavable. In some cases, the airway sensor(s) 110 is configured to transmit data indicative of the flow rate to the monitor 106, which uses the data to determine the inspiratory/expiratory volume, tidal volume, ventilation rate, inspiratory/expiratory time, or an amount of air leakage from the airway of the patient 104 at each ventilation cycle.

In various implementations, the airway sensor(s) 110 includes a pressure sensor configured to detect a pressure associated with the airway of the patient 104. For example, the pressure sensor includes an electrical circuit that includes a pressure transducer or pressure-sensitive resistor connected to the airway of the patient 104. The circuit generates an analog signal that is based on the pressure of the airway of the patient 104.

According to some examples, the airway sensor(s) 110 includes a CO₂ sensor, such as a nondispersive Infrared sensor (NDIR) sensor or spectrometer that uses spectroscopy to detect a concentration of CO₂ in the airway of the patient 104. Other types of CO2 sensors are optionally used, such as a photoacoustic sensor or a heteropolysiloxane-based sensor. In some cases, the airway sensor(s) 110 includes an O₂ sensor, such as zirconia sensor, an ultrasound sensor, a paramagnetic sensor, or a titanium sensor.

In various implementations, the monitor 106 and/or ventilation device 108 includes a repository configured to receive air expired from the airway of the patient 104. The repository, for example, is a bag or a box that is fluidically coupled to the airway of the patient 104. The repository is large enough to hold air from several expired breaths of the patient 104, in some cases. The repository mixes the gas it contains. For instance, the repository includes a fan or protrusions that cause turbulent flow within the interior of the reposiory, in some cases. In some implementations, the airway sensor(s) 110 are configured to detect the airway parameter(s) of the gas held in the repository. For instance, the airway sensor(s) 110 include a CO2 sensor and/or an O2 sensor configured to detect a partial pressure of CO2 and/or O2 in the air contained in the repository. Because the repository holds multiple breaths over an extended period of time, the airway sensor(s) 110 detecting airway parameter(s) in the repository may be configured to have a relatively long sampling period or low sampling rate. The measured parameters reflect average, or "mixed expired" gas measurements.

In some examples, an individual airway sensor 110 is configured to detect an airway parameter at a first location in the airway of the patient 104 and/or the ventilation device 108 as well as at a second location in the airway of the patient 104 and/or the ventilation device 108. For example, the individual airway sensor 110 is configured to detect the airway parameter in an endotracheal tube placed in the trachea of the patient 104 at a first sampling rate and the airway parameter in the expired air collected in the repository at a second sampling rate. The first sampling rate, for example, is greater than the second sampling rate.

In various implementations, the monitor 106 analyzes the airway parameter(s) detected by the airway sensor(s) 110. In addition, the monitor 106 may perform various secondary actions based on the airway parameter(s) detected by the airway sensor(s) 110. These actions include, for instance, outputting a signal to the rescuer 102, calculating a metric associated with the condition of the patient 104, triggering detection of another parameter of the patient 104, adjusting or correcting another parameter of the patient 104, or causing the ventilation device 108 to change at least one ventilation parameter.

For example, the monitor 106 may output signals to the rescuer 102. For instance, the monitor 106 includes a screen that visually outputs a signal, a speaker that audibly outputs a signal, a haptic device that outputs a signal as vibration, or a combination thereof. In various cases, the monitor 106 outputs one or more signals based on the airway parameter(s). In some cases, the monitor 106 stores data indicative of the airway parameter(s), which can be accessed at a later time for the purposes of post-event review. In some implementations, the monitor 106 and/or the airway sensor(s) 110 include a transceiver configured to transmit data indicative of the airway parameter(s) to an external device (e.g., a remote server) for storage, analysis, or playback for post-event review. In some cases, the monitor 106 outputs a signal to the ventilation device 108 based on the airway parameter(s), which may cause the ventilation device 108 to perform an action associated with administering the assisted ventilation to the patient 104.

According to some cases, the monitor 106 alarms based on the airway parameter(s). As used herein, the term "alarms," "alerts," and their equivalents, refers to a device selectively outputting a signal when a predetermined condition has been satisfied. For example, the monitor 106 may alarm when a particular airway parameter exceeds a first threshold and/or is less than a second threshold, and may refrain form alarming when the airway parameter is between the first threshold and the second threshold. In some implementations, the monitor 106 alarms by outputting a particular visual signal (e.g., a flashing light, a particular color, such as red, etc.), a particular auditory signal (e.g., a particular beep, siren, etc.), or by vibrating. In some cases, the monitor 106 alarms by outputting a message to the rescuer 102, such as a warning about a suspected condition of the patient, a warning about a suspected condition of the ventilation device 108, or an instruction (e.g., to perform an action to prevent deterioration of the condition of the patient 104; or to perform an action to adjust a suspected condition of the ventilation device 108, such as a leak). Various types of alarms will be described herein.

In various implementations, the monitor 106 monitors the type of breathing of the patient 104 based on the airway parameter(s). For instance, the rescuer 102 intubates the patient 104 and places an endotracheal tube, attached to the ventilation device 108, inside the trachea of the patient 104. In advance of placing the endotracheal tube, the rescuer 102 induces anesthesia by administering one or more medications, such as a paralytic and a sedative, to the patient 104. The paralytic and sedative enable the rescuer 102 to place the endotracheal tube without causing significant damage and/or discomfort to the patient 104. When active, the paralytic prevents the patient 104 from spontaneously breathing. The ventilation device 108 administers assisted ventilation to the patient 104 when the patient 104 can no longer breathe on their own. In some examples, the duration of action the paralytic and the sedative used for induction are roughly the same. For these regimens, by knowing when the paralytic wears off, the monitor 106 can infer when the sedative is wearing off. Since being intubated without sedation can be uncomfortable and traumatizing, redosing the patient 104 with sedation as soon as (if not sooner) the induction dose is wearing off is important.

In some cases, the monitor 106 determines a time at which the paralytic induces paralysis of the patient. For example, the monitor 106 may identify, based on the CO₂ waveform, a pattern indicative of diminishing exhalation waveforms (e.g., in response to determining that the paralytic has been administered to the patient 104). In some cases, the monitor 106 further determines the onset of paralysis based on the pattern of airway pressure of the patient 104. According to some examples, the monitor 106 identifies the paralysis by identifying a decreasing tidal volume over a period of time (e.g., 30 seconds) based on measurements from a flow sensor. In some examples, the monitor 106 calculates a variability index to determine whether the patient 104 has intermingling of spontaneous and PPV breaths. In some cases, the monitor 106 further relies on pressure measurements from a pressure sensor in order to distinguish between PPV breaths and spontaneous breaths, and may limit its analysis of decreasing tidal volume to the spontaneous breaths. For instance, breaths without positive pressure in the airway can be classified as spontaneous breaths. According to some examples, the monitor 106 may determine that the paralytic has induced paralysis upon determining that the patient 104 has not had a spontaneous breath for at least a time interval (e.g., a time interval that is greater than 4 seconds and less than 30 seconds). In various cases, the monitor 106 may output an indication of the paralysis. In some cases, this can cause the rescuer 102 to begin laryngoscopy and intubation of the patient 104. According to some implementations, the monitor 106 may store and/or transmit an indication of the time of the paralysis, which can be referred to during post-event review.

In various implementations, the monitor 106 detects one or more spontaneous breaths of the patient 104 based on the airway parameter(s). The patient 104 may be unable to breathe sufficiently, or at all, on their own. For example, the patient 104 is paralyzed with the paralytic or could be experiencing a drug overdose or traumatic brain injury (TBI) that causes the patient 104 to stop breathing. In these circumstances, the rescuer 102 may administer assisted ventilation to the patient 104 via the ventilation device 108.

However, in some circumstances, the patient 104 at least partially begins or increases breathing on their own during the rescue event. For example, the patient 104 may be recovering from their drug overdose or TBI. In some cases, the anesthesia (e.g., the paralytic) used to intubate the patient may be wearing off. In these cases, the patient 104 may produce one or more spontaneous breaths. Knowing when the patient 104 is producing spontaneous breaths can be valuable to the rescuer 102. For example, patients recovering from drug overdoses may unexpectedly react violently, which could be dangerous for the rescuer 102. By knowing that the patient 104 is recovering, the rescuer 102 can take steps to avoid injury. If the rescuer 102 identifies that the patient 104 is no longer paralyzed, then the rescuer 102 may know to redose the patient 104 with the sedative and paralytic before the patient 104 experiences significant discomfort due to the intubation. In some cases, the spontaneous breath can impact other factors tracked by the monitor 106 that the rescuer 102 relies upon to make certain clinical decisions. For example, the monitor 106 may instruct the rescuer 102 to ventilate the patient 104 at a particular rate and may adjust that instructed rate based on the presence of spontaneous breaths. In some implementations, the monitor 106 generates data indicating the transition between types of breaths (e.g., spontaneous breaths to PPV breaths, or vice versa). The monitor 106 may output indications of the transition to the rescuer 102. In some cases, the monitor 106 transmits the data to an external device for post-event review or real-time support by a clinician operating the external device and communicating with the rescuer 102 during the event.

In some cases, the monitor 106 may detect each breath of the patient 104 (e.g., based on the CO₂ waveform, or based on the airway pressure) and classify each breath as either a positive pressure ventilation (PPV) breath or a spontaneous breath. As used herein, the term "PPV breath," and its equivalents, may refer to a breath applied using assisted ventilation administered to a patient. In some examples, the monitor 106 distinguishes the PPV breaths from the spontaneous breaths based solely on the CO₂ waveform. In some cases, the monitor 106 uses another airway parameter to identify the different types of breaths. For example, the monitor 106 distinguishes PPV breaths and spontaneous breaths based on an airway flow rate waveform and/or a derived tidal volume of the patient 104. In some cases, spontaneous breaths (at least in patients receiving PPV in emergency care situations) have a different flow envelope and/or tidal volume than PPV breaths, which can be used by the monitor 106 to distinguish the types of breaths. In another example, the monitor 106 distinguishes PPV breaths and spontaneous breaths based on the airway pressure of the patient 104. Various techniques related to capnography analysis are described in U.S. Patent No. 10,820,833, which is hereby incorporated by reference. In other examples, the monitor 106 distinguishes PPV breaths and spontaneous breaths based on a combination of two or more airway parameters of the patient 104, such as an assessment of the presence or absence of a rise and fall in positive airway pressure temporally corresponding to an inspiratory phase of a CO₂ waveform. In various implementations, the monitor 106 may store and/or transmit an indication of the time that PPV breaths begin and/or end, which can be referred to during post-event review.

In various implementations, the monitor 106 may take one or more actions based on the detected spontaneous breath. In some cases, the monitor 106 outputs an indication of the spontaneous breath. In some implementations, the monitor 106 outputs a warning based on the spontaneous breath. According to some cases, the monitor 106 outputs an instruction based on the spontaneous breath. For example, the monitor 106 instructs the rescuer 102 to re-administer anesthesia to the patient 104 based on detecting the spontaneous breath.

In some implementations, the monitor 106 stores an indication of the spontaneous breath. For instance, the monitor 106 stores tags indicating whether each breath was a PPV breath or a spontaneous breath, such as time-stamps attributed to PPV breaths and/or spontaneous breaths. In some implementations, the monitor 106 determines and/or outputs a rate of PPV breaths administered to the patient 104, a rate of spontaneous breaths performed by the patient 104, a combined rate of total breaths of the patient 104, or any combination thereof. In some cases, the monitor 106 determines and/or outputs a trend indicative of the rate a rate of PPV breaths administered to the patient 104, the rate of spontaneous breaths performed by the patient 104, the combined rate of total breaths of the patient 104, or any combination thereof over time. In some cases, the monitor 106 alarms and/or outputs a warning based on the rate of PPV breaths administered to the patient 104 being outside of a first range, the rate of spontaneous breaths performed by the patient 104 being outside of a second range, the rate of combined rate of total breaths of the patient 104 being outside of a third range, or any combination thereof. Thus, in various implementations, the monitor 106 is configured to provide feedback to the rescuer 102 about the presence and/or frequency of spontaneous breaths.

In various cases, the monitor 106 infers a condition of the patient 104 based on one or more detected spontaneous breaths of the patient 104. In various examples, a predetermined pattern of spontaneous breaths is associated with brain damage. Examples of the predetermined pattern include, for example, cluster breathing (also referred to as "Biot's respirations"). Cluster breathing is characterized by irregular clusters of spontaneous breaths followed by apneic episodes of variable duration. Cluster breathing is associated with brainstem problems, such as upper medulla lesions and lower pons lesions. Thus, in some cases, the monitor 106 outputs a warning that the patient 104 has a suspected severe brain injury upon recognizing cluster breathing based on the airway parameter(s). Another example of the predetermined pattern includes Cheynes-Stokes breathing, which is characterized by cyclical episodes of apnea and hyperventilation. Cheynes-Stokes breathing is associated with brain injury from various causes, including heart failure and stroke. In some implementations, the monitor 106 outputs a warning that the patient 104 is exhibiting a Cheynes-Stokes breathing pattern. The monitor 106 may further output a warning that the patient 104 has suspected heart failure and/or stroke upon recognizing Cheynes-Stokes breathing based on the airway parameter(s). In some other implementations, the monitor 106 outputs a warning that the patient 104 is exhibiting an abnormal breathing pattern. The monitor 106 may additionally output information indicating which parameter(s) of breathing are exhibiting an abnormal pattern, and/or information indicative of time intervals associated with the abnormal breathing pattern.

In various cases, upon detecting the spontaneous breath(s) of the patient 104, the monitor 106 compares the pattern of the spontaneous breath(s) to the predetermined pattern. If the monitor 106 determines that the detected spontaneous breath(s) match the pattern, the monitor 106 determines that the patient 104 may be experiencing a condition associated with the pattern (e.g., traumatic brain injury, heart failure, stroke, or another associated condition). The monitor 106 stores and/or transmits an indication of the determination, or alarms based on the determination.

In some implementations, the monitor 106 may generate a metric based on the airway parameter(s). As used herein, the term "metric," "figure of merit," and their equivalents, can refer to a quantity indicative of a physiological condition of a patient, or a treatment administered to the patient, that is determined based on one or more underlying physiological parameters of the patient. Examples of metrics determined by the monitor 106 include PEEP, PIP, minute ventilation, a CO₂ extraction rate, airway leak fraction, a positive pressure fraction, a spontaneous breathing index, a variability index (e.g., a tidal volume variability index, or a rate variability index), a hypoxemia dose index, a desaturation severity index, and so on. According to some implementations, the monitor 106 transmits or otherwise outputs data indicative of sensor readings and/or physiological parameters to the external device, wherein the external device may generate the metric. In various implementations, the external device is located remotely from the location of the patient care event.

The monitor 106 determines the PEEP and/or PIP of the patient 104 based on the airway pressure detected by the airway sensor(s) 110. The monitor 106 may additionally utilize the airway flow and/or airway CO₂ detected by the airway sensor(s) 110 in the determination of PEEP and/or PIP of the patient 104. In various cases, the monitor 106 detects an expiratory event of the patient 104 based on the detected flow rate over time, or based on the detected CO₂ concentration or partial pressure over time, or based on the detected airway pressure over time. As used herein, the term "expiratory event" refers to air flowing out of a patient's airway without air flowing into the patient's airway. When the patient is spontaneously breathing, an expiratory event is equivalent to exhalation. The monitor 106 determines an end time of the expiratory event and determines the PEEP by identifying the pressure detected by the airway sensor(s) 110 at the end time of the expiratory event. In some implementations, the monitor 106 detects an inspiratory event of the patient 104 based on the detected flow rate over time, or based on the detected CO₂ concentration or partial pressure over time, or based on the detected airway pressure over time. As used herein, the term "inspiratory event" refers to air flowing into a patient's airway without air flowing out of the patient's airway. When the patient is spontaneously breathing, an inspiratory event is equivalent to inhalation. The monitor 106 determines a time interval of the inspiratory event (e.g., a time interval between the beginning of the inspiratory event and the end of the inspiratory event). The monitor 106 determines the PIP by determining a maximum pressure detected during the time interval of the inspiratory event. The monitor 106 performs one or more actions based on the PEEP and/or the PIP, such as outputting the PEEP and/or the PIP, storing the PEEP and/or the PIP, alarming if the PEEP and/or the PIP are outside of a particular range, or controlling the ventilation device 108 based on the PEEP and/or the PIP.

The monitor 106 determines the CO₂ extraction rate based on CO₂ measurements detected in the airway of the patient 104. For instance, the monitor 106 identifies the CO₂ level (e.g., detected using an on-airway CO₂ sensor or a sidestream sensor) in the mixed expired gas in the repository, and multiplies the CO₂ level by the minute ventilation. In some examples, the monitor 106 determines the CO₂ extraction rate by multiplying the airway CO₂ fraction (e.g., detected using an on-airway CO₂ sensor) and airway flow, and average that signal over time (e.g., with a slow enough average to smooth out individual breaths).

The monitor 106 determines the positive pressure fraction of the patient 104 based on the pressure detected by the airway sensor(s) 110. In various implementations, overventilation and/or hyperventilation produces excess pressure in the lungs of the patient 104. The excess pressure can impede venous return of blood to the heart, which can result in deterioration of the condition of the patient 104, or damage to the patient 104. Pressures that impede venous return are significantly lower than pressures that physically and directly damage the lungs of the patient 104. The monitor 106 may alarm based on the positive pressure fraction.

The monitor 106 determines the airway pressure of the patient 104 based on the pressure detected by the airway sensor(s) 110. In some examples, excess pressure in the lungs of the patient 104 can physically damage the lungs, particularly in cases in which the patient 104 is intubated and there is a closed fluid circuit between the lungs of the patient 104 and the ventilation device 108. In various implementations in which the ventilation device 108 is a bag-valve mask, excess pressure administered by the bag-valve mask can force air into the stomach of the patient 104. The patient 104 may then regurgitate their stomach contents, which can result in the stomach contents entering the airway or even the lungs, thereby causing significant damage to the patient 104. In some implementations, the ventilation device 108 itself is associated with a particular oropharyngeal leak pressure.

In some cases, the monitor 106 alarms when it determines that the airway pressure is above a particular threshold. In some cases, the threshold is predetermined or set by a user, such as the rescuer 102. In some implementations, the threshold is based on a type of the ventilation device 108, which can be identified based on a look-up table or other data stored by the monitor 106.

In various cases, the monitor 106 may alarm based on the positive pressure fraction. In some particular implementations, the airway sensor(s) 110 detect the pressure during a time interval. In various implementations, the monitor 106 determines the positive pressure fraction based on an amount of the time interval (e.g., a fraction or percentage of the time interval) at which the detected pressure exceeds a particular threshold. In some cases, the threshold is predetermined or set by a user, such as the rescuer 102. In some implementations, the threshold is based on a type of the ventilation device 108, which can be identified based on a look-up table or other data stored by the monitor 106. According to some implementations, the positive pressure fraction is equal to the amount of the time interval that the detected pressure exceeds the particular threshold. In some cases, the positive pressure fraction is based on a percentage or ratio of the time interval during which the detected pressure is within a desired range. According to some implementations, the positive pressure fraction is repeatedly and/or continuously determined by the monitor 106 based on a sliding time interval that ends at a current time.

The monitor 106 performs one or more actions based on the positive pressure fraction. In some implementations, the monitor 106 outputs the positive pressure fraction to the rescuer 102. For instance, the monitor 106 outputs a waveform indicative of the positive pressure fraction over time and/or a numerical indicator of the value of the metric at a given time. In some cases, the monitor 106 stores the metric and/or transmits data indicative of the positive pressure fraction to an external device for storage and post-event review. The rescuer 102 may use the positive pressure fraction to manually adjust care of the patient 104, such as a ventilation rate or ventilation pressure. In some cases, the monitor 106 outputs a signal to the ventilation device 108, which causes the ventilation device 108 to vent and/or adjust a ventilation parameter based on the positive pressure fraction.

In some examples, the monitor 106 is configured to alarm when the positive pressure fraction exceeds a threshold. In some cases, the monitor 106 outputs an instruction based on the metric, such as an instruction to slow down the ventilation rate of the patient 104, or to reduce the airway pressure, if the positive pressure fraction exceeds a threshold. According to some examples, the monitor 106 transmits, based on the positive pressure fraction, a signal to the ventilation device 108 that causes the ventilation device 108 to vent and/or adjust a ventilation parameter. In some cases, the monitor 106 triggers detection of another parameter (e.g., blood pressure) in response to determining that the positive pressure fraction exceeds the threshold.

In an example process, the monitor 106 establishes the threshold pressure, which could be preconfigured or user-configurable. The monitor 106 may identify airway pressure sampled over a time window (e.g., a 30 second time window), and may compare each pressure sample to a threshold. If the pressure exceeds the threshold during greater than a particular percentage of the time window, the monitor 106 generates an alert and/or stores an indication of an event (e.g., an "excessive positive pressure fraction event"). In various cases, the monitor 106 may perform this process repeatedly, using a sliding time window. The monitor 106 may output the changing percentage of the time window at which the pressure exceeds the threshold and/or the changing alert status, in various cases. In some implementations, the monitor 106 triggers a blood pressure measurement of the patient 104 when the alert is active.

According to some implementations, the monitor 106 determines a spontaneous breathing index of the patient 104 based on a CO₂ level, airway pressure, a frequency of spontaneous breaths, and/or a flow rate detected by the airway parameter(s) 110. As used herein, the term "spontaneity," and its equivalents, is a characteristic of breaths that are performed by a patient (also referred to as spontaneous respiratory activity (SRA)), rather than an assistive ventilation device. As used herein, the term "asynchrony" refers to simultaneous and/or intermingled PPV and spontaneous breaths. For example, there may be asynchrony when the patient 104 undegoes an advanced airway management procedure, such as intubation, but is not pharmaceutically paralyed. Similarly, there may be asynchrony when the patient 104 undegoes an advanced airway management procedure, such as intubation, and the effect of an initial paralytic drug has worn off. In this circumstance the patient 104 may perform spontaneous breaths during the same time period that the rescuer 102 is delivering PPV. As described above, the monitor 106 is configured to detect whether breaths of the patient 104 are PPV breaths or spontaneous breaths. The monitor 106, in some cases, further determines the spontaneous breathing index based on the spontaneous breaths of the patient. In some cases, the spontaneous breathing index is a metric that represents the presence of spontaneous breaths in the breathing of the patient 104. For instance, the spontaneous breathing index is based on (e.g., equal to) a relative contribution of spontaneous breaths to the total breathing of the patient 104 (e.g., within a time interval), a number of spontaneous breaths (e.g., within a time interval), a CO2 contribution of the spontaneous breaths (e.g., versus PPV breaths), a rate (e.g., frequency) of the spontaneous breaths, a tidal volume of the spontaneous breaths, a minute volume of the spontaneous breaths, a density over time of the spontaneous breaths, a rate of change of any of these factors, or any combination thereof. In various cases, the monitor 106 may determine that there is a transition between the patient 104 spontaneous breathing and not spontaneous breathing by comparing the spontaneous breathing index to one or more thresholds.

In related implementations, the monitor 106 relies on the identified spontaneous breaths in order to filter or otherwise modify an airway parameter for further analysis. For example, by subtracting or otherwise removing the contribution of spontaneous breaths from an airway parameter waveform (e.g., a CO2 waveform, a pressure waveform, a flow rate waveform, etc.), the monitor 106 can rely on the waveform to reflect conditions of the assisted ventilation provided by the ventilation device 108. For example, the monitor 106 relies on the resultant waveform to identify the respiration rate and/or tidal volume contribution from the ventilation device 108 to the condition of the patient 104. In some cases, the monitor 106 identifies overlapping spontaneous and PPV breaths in the airway parameter waveform. The monitor 106 refrains from identifying a parameter and/or metric based on the overlapping spontaneous and PPV breaths. For example, if a CO2 waveform includes an overlapping spontaneous and PPV breath event, the monitor 106 refrains from identifying or reporting an EtCO2 value based on the CO2 waveform during the breath event.

In some cases, the monitor 106 may calculate a variability index based on the airway parameter(s). A variability index is based on the variance of an airway parameter (e.g., ventilation rate, airway pressure, per-breath EtCO2, tidal volume, etc.) during a time interval. For example, the monitor 106 determines a tidal volume variability index of the patient 104 based on the variance of tidal volumes detected across multiple breath events during a predetermined time interval. The tidal volume, for example, is calculated by the monitor 106 based on the flow rate detected by the airway sensor(s) 110. Because PPV breaths and spontaneous breaths are associated with different airway parameter characteristics (e.g., different EtCO2 levels, different tidal volumes, etc.), the variability index is also indicative of asynchrony. Thus, the monitor 106 is configured to detect spontaneous breathing and/or asynchrony of the patient 104 based on the variability index, in some cases. In some implementations, the variability index is also indicative of quality of ventilation administered to the patient 104, an airway leak, and other issues. For instance, the monitor 106 may determine that an issue (e.g., intermingling PPV and spontaneous breaths, poor quality ventilation, airway leak, etc.) is present by determining that the variability index is greater than a threshold.

By understanding asynchrony in the breaths of the patient 104, the monitor 106 may infer whether the patient may be in need of further anesthesia, without knowing what or when anesthesia was administered to the patient 104. In various implementations, the monitor 106 alarms and/or outputs a signal to the rescuer 102 based on the spontaneous breathing index and/or the tidal volume variability index. Accordingly, the rescuer 102 is informed of changes in the spontaneous breathing index and/or the tidal volume variability index and in turn informed of when to expect the patient 104 to regain consciousness and/or instructed to re-administer anesthesia to the patient 104.

In some implementations, the monitor 106 evaluates other metrics based on the type of breaths detected. For example, the monitor 106 may detect one or more airway parameters associated with PPV breaths as well as one or more airway parameters associated with spontaneous breaths. In some implementations, the spontaneous breaths may become strong enough to interfere with assisted ventilation. Accordingly, the monitor 106 may output a warning or alarm based on the airway parameter(s) associated with the spontaneous breaths being outside of a particular range, which may indicate that the spontaneous breaths are interfering with assisted ventilation. The warning or alarm, in some cases, may indicate to the rescuer 102 that the status of the patient 104 has changed.

In some implementations, the monitor 106 may determine when the paralytic and/or sedative is wearing off based on the metrics and/or presence of spontaneous breaths and output a warning to the rescuer 102. The rescuer 102, in turn, may redose the patient 104 with the sedative and/or paralytic, and/or administer another medication such as an analgesic, based on the warning. In some cases, the monitor 106 may store or transmit an indication of the spontaneous breathing to an external device, which may enable post-event review of the spontaneous breathing event.

In some implementations, the monitor 106 determines the hyperventilation index of the patient 104. When the patient 104 is hyperventilated (e.g., during assisted ventilation), the EtCO2 of the patient 104 decreases and the respiration rate increases. In various cases, the hyperventilation index is based on the amount of a time interval at which both an EtCO2 of the patient 104 is less than a first threshold and also that a respiration rate of the patient exceeds a second threshold. For example, the monitor 106 identifies a pair of EtCO2 and respiration rate values that are detected during in the same sub-interval. If the monitor 106 determines that both the EtCO2 value is below the first threshold and that the respiration rate is above the second threshold, the monitor 106 marks the sub-interval as a hyperventilated interval. The monitor 106, for example, determines the hyperventilation index by dividing the sum of the lengths of all hyperventilated intervals in a time interval by the total length of the time interval. The hyperventilation index is indicative of a burden of hyperventilating the patient 104 during assisted ventilation. The monitor 106 identifies the EtCO2 and respiration rate of the patient 104 based on signals from the airway sensor(s) 110. In some cases, the monitor 106 calculates the hyperventilation index repeatedly and/or periodically based on a sliding time window, and determines the magnitude of deviations (e.g., when the hyperventilation index arises above the threshold) and/or area under the curve of the hyperventilation index over time. In some cases, the monitor further relies on the CO₂ waveform in order to avoid relying on erroneous EtCO2 values (discussed below) for the purpose of determining the hyperventilation index. In some cases, the metric is calculated based on variable and/or nonlinear weighting in terms of time and severity of the hyperventilation index. For example, the contribution of a hyperventilation interval in which the respiration rate is extremely high is greater than the contribution of a hyperventilation interval in which the respiration rate is marginally elevated. According to some cases, the monitor 106 outputs the metric and/or otherwise provide feedback to the rescuer 102 so that the rescuer 102 can adjust assisted ventilation provided to the patient 104 to prevent hyperventilation.

In various implementations, the monitor 106 may alert the rescuer 102 that the patient 104 is likely being hyperventilated when the hyperventilation index is above a threshold. Hyperventilation may impede blood flow return to the heart of the patient 102, which can reduce blood pressure. Accordingly, the monitor 106 may automatically trigger a blood pressure measurement of the patient 102 in response to determining that the patient 102 is likely being hyperventilated. In some cases, the monitor 106 generates data indicating a time at which hyperventilation begins and/or ends. The monitor 106 may output the data to the rescuer 102 and/or transmit the data to an external device.

According to some cases, the monitor 106 may determine the hypoxemia dose index (also referred to as a "desaturation severity index") of the patient 104 based on SpO₂, ECG, and/or blood pressure of the patient 104. The hypoxemia dose index indicates hypoxemia of the patient 104 during a particular time interval. During an RSI process, there is a substantial danger of hypoxemia during the time interval between the time at which anesthesia (e.g., the paralytic and sedative) is administered to the patient 104 and the time at which assisted ventilation begins. During this time interval, the patient 104 stops breathing, the endotracheal tube is placed, and the ventilation device 108 begins administering PPV breaths to the patient 104. In various implementations, the monitor 106 identifies this time interval (also referred to as a "peri-intubation interval") based on the airway parameter(s) detected by the airway sensor(s) 110. For example, the monitor 106 identifies the start time of the time interval by detecting the absence of spontaneous breaths of the patient 104 and identifies the end time of the time interval by detecting the presence of PPV breaths administered to the patient 104. The monitor 106, in some examples, determines the hypoxemia dose index based on the ECG and/or SpO₂ of the patient 104 during the peri-intubation interval. The hypoxemia dose, for example, is negatively correlated with the SpO₂ of the patient 104. In some cases, the monitor 106 adjusts the hypoxemia dose index based on the heart rate of the patient 104, when the heart rate indicates a bradycardic response (e.g., determined based on pulse rate from an SpO₂ sensor and/or heart rate from ECG). In some implementations, the monitor 106 adjusts the hypoxemia dose index based on a physiologic condition of the patient 104, such as based on detection of hypotension (e.g., based on a blood pressure detected from the patient 104). In some implementations, the monitor 106 adjusts the hypoxemia dose inde based on a characteristic of the patient 104, such as an age of the patient 104, or a medical condition and/or previous diagnosis of the patient 104.

In some instances, the monitor 106 is configured to determine whether a first airway parameter is detected accurately based on the first airway parameter and/or a second airway parameter. In some implementations, the airway sensor(s) 110 are configured to detect an amount of CO₂ (e.g., a partial pressure of CO₂) in an airway of the patient 104 and/or within a duct of the ventilation device 108 that is connected to an airway of the patient 104. In various implementations, the monitor 106 generates a waveform indicative of the CO₂ level over time. In some cases, the airway sensor(s) 110 also detect another airway parameter, such as flow rate and/or airway pressure, in the same duct and/or fluidic circuit at which the CO₂ is detected.

In general, as long as the airway sensor(s) 110 are accurately detecting airway CO₂ of the patient 104 that is spontaneously breathing or being properly ventilated by the ventilation device 108, each breath event (e.g., inspiration and expiration) appears in the waveform in a particular, trapezoidal shape. For example, in a first phase of the breath event, the waveform remains at a negligible level (e.g., equal to 0 or below a particular threshold). In a second phase, the CO₂ level rises from a negligible level at a relatively high rate of change. In a third phase (also referred to as a "plateau phase") of the breath event, the waveform plateaus and/or rises at a relatively low rate of change. Finally, in a fourth phase of the breath event (also referred to as "phase 0"), the waveform decreases at a relatively high rate of change. This waveform shape is generally expected to repeat for each breath that is administered to the patient 104 by ventilation and/or each breath that is taken by the patient 104. The end tidal CO₂ (EtCO₂) of the patient 104 is measured at the peak of the waveform shape, which generally occurs at the end of the third (plateau) phase. In various implementations, the monitor 106 outputs the waveform and/or the EtCO₂ of the patient 104.

The EtCO₂ is a significant parameter for monitoring the condition of the patient 104, in various implementations. In some examples, the rescuer 102 uses the EtCO₂ to adjust a rate at which ventilated breaths are administered to the patient 104. If the EtCO₂ is higher than a first threshold, it may indicate that the patient 104 is being hypoventilated, and the rescuer 102 may increase the ventilation rate. If the EtCO₂ is lower than a second threshold, it may indicate that the patient 104 is being hyperventilated, and the rescuer 102 may decrease the ventilation rate. In some cases, the rescuer 102 may use the EtCO₂ as a metric for determining whether to continue treating the patient 104. For example, the length of time at which the patient 104 is receiving rescue care (e.g., cardiopulmonary resuscitation (CPR), assisted ventilation, etc.) and the EtCO₂ of the patient 104 may correlate to the likelihood that the patient 104 will recover. Thus, the rescuer 102 may have reason to stop administering rescue care if the duration of the rescue exceeds a first threshold time and the EtCO₂ of the patient 104 is below a second threshold.

However, in some cases, the EtCO₂ output by the monitor 106 does not correspond to the CO₂ level in the patient's 104 airway (e.g., lungs). For example, there may be a leak between a duct in which the airway sensor(s) 110 are coupled and the airway of the patient 104. In some cases in which the airway sensor(s) 110 are integrated into and/or attached to a bag-valve mask (e.g., included in the ventilation device 108), a leak can arise between the mask of the bag-valve mask and the face of the patient 104. In some cases, there is insufficient inflation pressure on the cuff of an endotracheal tube (e.g., a cuff that couples the endotracheal tube to the interior of the trachea of the patient 104 when inflated) that results in erroneously low CO₂ readings by the airway sensor(s) 110. Other problems include issues with the airway sensor(s) 110 itself, a problem with a duct attached to the airway sensor(s) 110, a problem with a valve attached to the duct, a problem created by another treatment (e.g., CPR, ventilation techniques, etc.) being provided to the patient, or the like. In general, these discrepancies may result in an erroneously low CO₂ level detected by the airway sensor(s) 110, and also an erroneously low EtCO₂ level output by the monitor 106. For example, an endotracheal tube or supraglottic airway device of the ventilation device 108 has migrated or become dislodged from the airway of the patient 104. For another example, the mask of a bag-valve-mask (which is serving as ventilation device 108) has not been fully sealed against the face of the patient 104 while PPV is being administered. For another example, CPR chest compressions being administered to the patient have produced oscillations in the CO₂ level at the site where the airway sensor(s) 110 measure CO₂. Due to these and other problems, the CO₂ level detected by the airway sensor(s) 110 is inaccurate (meaning that the CO₂ level detected by the airway sensor(s) 110 does not correspond to the CO₂ level in the lungs of patient 104), thereby leading to an erroneously low EtCO₂ level detected by the monitor 106. The erroneously low EtCO₂ level output by the monitor 106 may cause the rescuer 102 to deliver care or treatment to the patient 104 in a suboptimal or inappropriate manner, such as by hypoventilating the patient 104 and/or refraining from administering life-saving care to the patient 104. Leaks and problems with the duct and/or valve are also associated with erroneously low flow rates and/or tidal volumes detected by airway sensor(s) 110 in a fluidic circuit including the airway of the patient 104 and/or the ventilation device 108.

In various implementations described herein, the monitor 106 is configured to detect a problem that results in an artificially low airway parameter reading. In particular examples, the monitor 106 detects the problem by analyzing the CO₂ waveform detected by the airway sensor(s) 110. In particular, the monitor 106 can detect the problem by determining that a breath event in the waveform lacks the third phase (the plateau phase). In some cases, the absence of the plateau phase is indicative of an airway leak and/or inaccuracy in another airway parameter. For example, the monitor 106 may determine that the spectral signature (e.g., frequency domain representation) of a particular breath event differs from a spectral signature of a standard breath event. In some cases, the monitor 106 detects the problem by determining that the duration of the third phase is shorter than a threshold duration, or that the slope of the fourth phase is insufficiently steep, or that the duration of the fourth phase is greater than a threshold duration.

In some implementations, the monitor 106 detects the problem based on other airway parameters. For example, in various implementations, the monitor 106 is configured to determine, based on a flow rate and/or tidal volume of the patient 104, whether the returned (e.g., exhaled) tidal volume is smaller than the delivered (e.g., inhaled) tidal volume. For instance, the monitor 106 determines whether a difference between the delivered tidal volume and the returned tidal volume is above a threshold. This difference is indicative of air that has escaped the fluidic circuit including airway and the ventilation device 108.

In some cases, the airway sensor(s) 110 detect a pressure in the airway of the patient 104 or in a duct connected to the airway of the patient 104, and may detect the problem based on the pressure. In some cases, the monitor 106 may identify the problem based on a cross-correlation between the CO₂ waveform and the airflow and/or pressure.

The monitor 106 performs one or more actions based on the detected problem. In some implementations, the monitor 106 outputs a warning indicating the problem. In some cases, the monitor 106 outputs an indication that the EtCO₂, the flow rate, or tidal volume output by the monitor 106 is, or may possibly be, erroneously low. The rescuer 102, in turn, can adjust their reliance on the EtCO₂ and/or tidal volume based on these warnings and indications. In some cases, the monitor 106 refrains from analyzing, outputting, or transmitting the EtCO₂, the flow rate, or the tidal volume for other alarms and/or metrics based on identifying the problem. According to some examples, the monitor 106 outputs the airway parameter to the rescuer 102 (or in data transmitted to an external device) with a warning that the parameter may be inaccurate.

In some implementations, the monitor 106 outputs an instruction based on the detected problem. In some implementations, the monitor 106 outputs an instruction to check for leaks, such as leaks between a bag-valve mask and the face of the patient 104. According to some cases, the monitor 106 outputs an instruction to check for other types of problems, such as to check the inflation pressure on the cuff of the endotracheal tube or SGA, or to check whether the airway device has migrated or become dislodged. In some cases, the monitor 106 outputs an instruction to use two hands to hold down the bag-valve mask on the face of the patient 104, which can address any leaks that are present. According to some cases, the monitor 106 outputs an instruction to use a secondary, non-airway parameter (e.g., SpO₂) for reliance on setting the ventilation rate, deciding whether to withdraw care, or other clinical decisions.

In some implementations, the monitor 106 triggers a blood pressure measurement based on any of the airway parameter(s) and/or metrics described above. For example, the monitor 106 includes, or is communicatively coupled with, a blood pressure sensor (e.g., an invasive, catheter-based blood pressure sensor inserted into the patient 104 or a non-invasive blood pressure (NIBP) sensor, such as an ultrasound-based blood pressure sensor or a blood pressure cuff, such as used in an oscillometric blood pressure device). In some implementations, the monitor 106 causes the blood pressure sensor to detect the blood pressure of the patient 104 in response to the airway parameter(s) and/or metric being above a first threshold and/or below a second threshold. For instance, the monitor 106 triggers the blood pressure measurement in response to determining that the positive pressure ratio exceeds a threshold, or in response to identifying a desaturation or bradycardia event of the patient 104.

In various implementations described herein, the monitor 106 may be configured to detect a problem wherein exhaled CO₂ is being rebreathed by the patient 104 during subsequent breaths. For example, the monitor 106 may make this determination based on analysis of the slope of the fourth phase (e.g., the inspiratory downstroke) of the CO₂ waveform, and/or based on the lowest CO₂ level(s) reached during the first phase (e.g., the inspiratory baseline). The monitor 106 performs one or more actions based on the detected problem. In some implementations, the monitor 106 outputs a warning indicating the problem, and/or outputs an instruction based on the detected problem. According to some implementations, the detection of the problem, and/or the action based on the detected problem, is performed by an external device based on data it receives from the monitor 106. For instance, the data is indicative of sensor readings and/or physiological parameters generated by the monitor 106. In some cases, the cause of the rebreathing problem is that additional deadspace has been added to the airway between the location where CO₂ is being sampled, and the location where expired gas is exhausted and fresh gas is introduced. For example, one or more devices such as a HEPA filter, an adapter for an airway sensor, or nebulizer tubing may have been added to the airway by the rescuer 102. The additional deadspace created by the added devices may cause rebreathing of exhaled CO₂. Accordingly, in some implementations, the monitor 106 outputs a warning indicating that additional deadspace in the airway has been detected, or the monitor 106 may output an instruction based on the detection of additional deadspace. For example, the monitor 106 indicates to the rescuer 102 that elevated EtCO₂ levels may be a result of deadspace which has been added to the airway.

In some cases, the airway sensor(s) 110 detect the partial pressure of CO₂ in the airway of the patient 104 and/or the EtCO₂ of the patient 104. The monitor 106 may, in some cases, further determine a CO₂ extraction (in units of volume per time interval) of the patient based on the partial pressure of CO₂ and/or the EtCO₂ of the patient 104. According to some cases, the airway sensor(s) 110 detect an airway pressure of the patient 104 over time. In various implementations, the monitor 106 can identify the PEEP level of the patient based on the airway pressure. In various implementations, the monitor 106 outputs the CO₂ extraction and/or the PEEP level. In some cases, the monitor 106 transmits data indicating the CO₂ extraction and/or the PEEP level to an external device, such as in a data stream.

In some implementations, the monitor 104 identifies a plateau pressure of the airway of the patient 104. For example, the airway sensor(s) 110 may include a pressure sensor configured to detect a pressure of the airway of the patient 104. In some cases, the monitor 106 determines a plateau pressure and/or peak pressure based on the airway pressure over time. In various implementations, plateau pressure represents a pressure in the alveoli of the patient 104. In some cases, the monitor 106 outputs the plateau pressure and/or peak pressure to the rescuer 102.

According to some implementations, the monitor 106 may differentiate between decreased lung compliance (in which both peak and plateau pressures are elevated) and increased resistance (which is associated with elevated peak pressure but non-elevated or normal plateau pressure). Decreased lung compliance may be associated with conditions such as pneumonia, adult respiratory distress syndrome (ARDS), and pulmonary edema. Accordingly, the monitor 106 may be configured to identify a condition of the patient 104 based on the peak and plateau pressures. Increased airway resistance can be associated with bronchospasm, a kink or partial obstruction of a duct in the ventilation device 108, or other issue. In some cases, the monitor 106 may be configured to identify that these issues based on the increased airway resistance. According to various implementations, the monitor 106 may output warnings or instructions for treating these conditions and/or issues to the rescuer 102.

In some implementations, the airway sensor(s) 110 detects a flow rate of air flowing through the airway of the patient 104. The monitor 106 may determine a tidal volume of the patient 104 based on the flow rate. In various implementations, the tidal volume of the patient 104 may be correlated with, or associated with, the corresponding plateau pressure of the patient 104 at that time. For example, the monitor 106 may visually display a waveform showing plateau pressure measurements and delivered tidal volume (optionally filtering out measurements associated with sufficiently, below-average tidal volumes), which can convey (to the rescuer) the order at which the various parameters were detected. In some implementations, the monitor 106 may monitor the plateau pressure based on the CO₂ waveform.

In a particular example, the monitor 106 samples the direction and magnitude of airflow through the airway of the patient 104. Optionally, the monitor 106 further identifies the CO₂ waveform of the patient. The monitor 106 may identify a transition from positive airflow (inward to the lungs of the patient 104) to zero flow. As a particular time elapses after the transition (e.g., 0.5 to 2 seconds), and before negative (outward from the lungs of the patient 104) airflow begins, the monitor 106 notes the instantaneous airway pressure of the patient 104. This instantaneous pressure may be logged as an plateau pressure measurement. If negative airflow begins before the particular time elapses after the transition, then the opportunistic plateau pressure is not obtained. In some examples, the contemporaneous tidal volume is also noted with the plateau pressure measurement. Accordingly, the monitor 106 and/or the rescuer 102 avoids comparing plateau pressure measurements associated with vastly different tidal volumes.

In various cases, the monitor 106 may perform one or more actions based on the plateau pressure. For example, the monitor 106 could store an indication of the plateau pressure or transmit (e.g., stream) data indicative of the plateau pressure and the tidal volume to an external device. In some cases, the monitor 106 outputs the plateau pressure with the tidal volume. According to some implementations, the monitor 106 triggers an alarm if the plateau pressure is higher than a threshold. In various examples, the monitor 106 stores and/or displays a measurement of lung compliance, which is determined by dividing the tidal volume by the plateau pressure. In some cases, the monitor 106 compares the lung compliance value to a previous measurement of compliance, or displays a graph of lung compliance over time, as a way to reveal changes in lung compliance.

In various examples, the monitor 106 may further include a sensor configured to detect ambient pressure. In cases where the monitor 106 determines or identifies that the patient 104 has a particular condition (e.g., a cuffed airway device, pneumothorax, or the like, which can be detected based on the CO₂ waveform, or based on interaction with or input into the monitor 106 by rescuer 102), then the monitor 106 detects that there is a change in altitude based on the ambient pressure (e.g., the patient 104 is being transported in an aircraft that has changed altitude), and output a corresponding alert. The change in altitude and/or pressure changes the pressure of enclosed fluidic circuits, such as the inflated cuff sealing the endotracheal tube to the airway of the patient 104 or the airway of the patient 104 itself, if it is sealed with the ventilation device 108. The alert, for example, may include an instruction to check the cuff or adjust the pressure in the ventilation device 108 and/or airway of the patient 104 to avoid injury resulting from the change in altitude.

In some implementations, the monitor 106 detects whether an endotracheal tube of the ventilation device 108 has been erroneously placed in the esophagus of the patient 104 rather than the trachea. For example, the monitor 106 determines that the ventilation device 108 is administering air to the patient 108 without a corresponding increase in another parameter of the patient 104, such as without a corresponding increase in EtCO₂, SpO₂, rSO₂, or the like. The monitor 106, in various implementations, alerts based on this determination. Accordingly, the rescuer 102 is informed that the endotracheal tube should be removed and correctly placed in the trachea of the patient 104.

According to various examples, the monitor 106 detects right mainstem intubation of the patient 104. Right mainstem intubation is a complication in which a tube (e.g., the endotracheal tube) is erroneously pushed through the trachea of the patient 104 and into the right main bronchus of the patient 104. The trachea branches into the right and left main bronchi, which connect to the lungs. Because the left main bronchus is typically narrower than the right, and branches from the trachea at a greater angle than the right, right mainstem intubation is significantly more common than left mainstem intubation. Right mainstem intubation is problematic for the patient, because it limits the supply of assisted ventilation to a single lung of the patient 104. Thus, recognizing right mainstem intubation is important, particularly in RSI procedures.

In various examples, the monitor 106 identifies the airway pressure and tidal volume of the patient 104 based on the airway parameter(s) detected by the airway sensor(s) 110. In some cases, the monitor 106 detects right mainstem intubation by detecting an increase in airway pressure. In various cases, the monitor 106 detects right mainstem intubation by detecting a relatively high pressure for a given tidal volume. In some cases, the monitor 106 also relies on a height and/or body weight of the patient 104 in order to detect the right mainstem intubation. For example, the rescuer 102 inputs the height and/or body weight of the patient 104 into the monitor 106. The monitor 106 outputs an indication of the suspected right mainstem intubation, in various cases.

In some cases, the monitor 106 receives an indication of a time of an event from the rescuer 102. The monitor 106 detects whether that time is erroneous by comparing the time to times of various parameters detected from the patient 104. For example, the monitor 106 infers that an entered time is incorrect based on a physiologic waveform and/or trend data detected by the monitor 106 at the time. In particular examples, the monitor 106 determines that an entered time of an intubation event of the patient 104 is incorrect if the CO₂ waveform before and after the entered time indicates continuous breathing by the patient 104. For instance, the monitor 106 receives an input signal from the rescuer 102 indicating a reported time at which an intubation tube is inserted into the airway of the patient 104. If the monitor 106 determines that an airway parameter waveform (e.g., a CO₂ waveform) detected during the reported time indicates continuous breathing, the monitor 106 may determine that the reported time is erroneous, because the patient 104 could not have continuous breaths during insertion of the intubation tube. In addition, if the monitor 106 determines that the airway parameter waveform is at 0 within a threshold time period overlapping and after the reported time, then the monitor 106 may also determine that the reported time was erroneous. In various cases, the monitor 106 refrains from storing or transmitting an entered time that is erroneous. In other cases, the monitor 106 stores or transmits an indication that an entered time is erroneous, in conjunction with storing or transmitting the entered time. According to some implementations, the monitor 106 selectively stores and/or transmits entered times that the monitor 106 confirms are feasible.

According to other implementations, the monitor 106 selectively stores and/or transmits metrics derived from the entered times, after the monitor 106 confirms that the entered times are feasible. In some cases, the detection of an erroneous event time provided by the rescuer 102 is performed by an external device that receives data indicating one or more relevant physiological parameters from the monitor 106. According to some implementations, the event time provided by the rescuer 102 may be part of the data transmitted from the monitor 106. In other implementations, the event time provided by the rescuer 102 is derived from a different source, such as an electronic patient care record. In some cases, the rescuer 102 enters or documents the event time at a first time point, and the detection of the erroneous event time provided by the rescuer 102 is performed at a later second time point.

Various other conditions are detected by the monitor 106, in various examples. For instance, the monitor 106 detects dynamic hyperinflation of the patient 106 based on the flow rate detected by the airway sensor(s) 110. For instance, if the monitor 106 detects (based on the flow rate) that inspiratory flow begins before the expiratory flow of a previous breathing event reaches zero, the gas volume in the lungs of the patient 104 will increase over time, thereby leading to dynamic hyperinflation. Based on detecting this condition, the monitor 106 outputs an instruction to the rescuer 102, or a signal to the ventilation device 108, to alter the respiration rate of the assisted ventilation administered to the patient 104, or to take some other action to relieve the dynamic hyperinflation condition. In some cases, the monitor 106 uses the airway parameter(s) and/or other parameters of the patient 104 to detect very low lung compliance (e.g., atelectasis), change in lung compliance, tensioning pneumothorax, very low cardiac output, poor ventilation and CPR coordination, excessive tidal volume, or the like.

In some cases, the monitor 106 outputs instructions that provide guidance on treating the patient 104 based on the airway parameter(s) and/or other parameters of the patient 104. For example, in some cases, the monitor 106 outputs an instruction indicating when to administer chest compressions to the patient during CPR based on the airway parameter(s) and/or other parameters. In some cases, the monitor 106 outputs an instruction indicating ventilation timing (e.g., when to time inspiration, when to time expiration, an ideal duration of inspiration, etc.) based on the airway parameter(s) and/or other parameters. Accordingly, the rescuer 102 receives real-time guidance from the monitor 106 on how to administer therapy to the patient 104 in order to optimize the condition of the patient 104.

In various implementations, the monitor 106 assesses pre-oxygenation adequacy of the patient 104 based on the airway parameter(s). As used herein, the term "pre-oxygenation," and its equivalents, refers to the administration of oxygen to a patient prior to induction of anesthesia in order to maximize the blood oxygenation and to maximize the concentration of oxygen in the lungs (also referred to as "denitrogenation") of the patient 104 prior to performing RSI. After induction and before the ventilation device 108 is placed and actively performing assisted ventilation on the patient 104, the patient 104 is not receiving oxygen, since the patient 104 can no longer breathe on their own and the ventilation device 108 is not yet administering PPV breaths to the patient 104. Accordingly, pre-oxygenation enables the rescuer 102 to prepare the patient 104 for an apneic event that occurs during intubation.

In various cases, the rescuer 102 applies a bag-valve mask to the patient 104, which supplies oxygenated air to the patient. The airway sensor(s) 110, for example, monitor pressure in the bag-valve mask. The monitor 106 determines whether there is a leak between the bag-valve mask and the patient 104 based on the airway parameter(s). For example, the monitor 106 determines whether there is a leak based on a detected pressure in the bag-valve mask, which corresponds to the pressure of oxygen in the mask that is being administered to the airway of the patient 104 during pre-oxygenation. If the pressure falls below a threshold, then the monitor 106 determines that there is a potential leak in the bag-valve mask. In some examples, the monitor 106 outputs an indication of the detected leak to the rescuer 102.

In some implementations, the monitor 106 assesses pre-oxygenation adequacy by determining whether the pressure in the bag-valve mask (e.g., continuously) exceeds a threshold pressure for greater than a threshold amount of time. The monitor 106, for example, outputs an indication that the pre-oxygenation is adequate when the pressure exceeds the threshold pressure for greater than the threshold amount of time. In some cases, the monitor 106 may recognize that the patient 104 is safe to intubate upon perceiving the indication. If the pressure drops below the threshold, then the monitor 106 alerts the rescuer 102. In some examples, the monitor 106 further uses EtO₂ (e.g., an oxygen level detected at the end of an expiratory event) of the patient 104 to assess denitrogenation of the patient 104 during pre-oxygenation. For instance, the monitor 106 analyzes the trajectory or trend of EtO₂ during the pre-oxygenation phase to provide real-time feedback about whether the pre-oxygenation strategy is working on the patient 104.

In various implementations described herein, the monitor 106 performs one or more actions based on at least one airway parameter detected by the airway sensor(s) 110. In some cases, the monitor 106 outputs and/or stores data indicative of the airway parameter(s) or an analysis of the airway parameter(s) (e.g., a metric or condition identified based on the airway parameter(s). In some implementations, the monitor 106 transmits a signal indicative of the data to an external device, such as an external computing device. In some implementations, the external device stores and/or outputs the data to a user. In some implementations, the external device calculates or otherwise generates a metric (such as any of the various metrics described herein) based on the airway parameter data transmitted from the monitor 106. The user, for example, accesses the data as part of a post-event review process. For instance, the user evaluates the quality of care administered to the patient 104 and/or performs an independent assessment of the condition of the patient 104 using the data output by the external computing device.

In particular examples, the monitor 106 and the airway sensor(s) 110 are combined into a standalone device. For example, the standalone device is carried or worn by the rescuer 102. In some cases, the standalone device also includes the ventilation device 108. For instance, the standalone device is a smart bag-valve mask. The standalone device detects airway parameter(s) of the patient 104, stores data based on the airway parameter(s), and optionally provides feedback to the rescuer 102 during a first time interval. At a later time (e.g., during a second later time interval), the standalone device transfers (e.g. wirelessly) its recorded data to a second device that has arrived on scene. For example, the standalone device wirelessly transmits a signal indicative of the data to a monitor-defibrillator. In this way, the monitor-defibrillator can gain awareness of airway management and/or ventilation care delivered before the monitor-defibrillator arrives on scene, and can integrate that "pre-arrival" data and knowledge into its own stored data record, and potentially into metrics and graphical information it presents on the monitor screen or in the form of alerts.

In particular implementations, the airway sensor(s) 110 are integrated into a standalone device. For example, the device is configured ot removably attach to the ventilation device 108 in order to monitor the airway parameter(s). Optionally, the device is disposable. In some cases, the device includes a flow sensor, a HEPA filter, an airway CO₂ sensor, an airway pressure sensor, or any combination thereof. In some cases, the HEPA filter is dual-purpose and is configured ot both filter air traveling through the ventilation device 108 and a resistive element across which the pressure drop is detected to measure airway flow. The airway CO2 sensor is a mainstream or a sidestream sensor. For example, a mainstream CO2 sensor is either an on-airway sensor (e.g., an infrared sensor including an infrared source, an optical path, and an infrared detector), and includes a cleanable and/or disposable tube with a window suitable for an accurate infrared-based measurement. In some instances, a sidestream CO2 sensor includes a sampling tube through which an air sample is syphoned from the airway of the patient 104 to a capnograph sensor. The airway pressure sensor is, for example, an air-filed tube attached to a durable pressure transducer, a disposable pressure sensor integrated with a duct attached to the airway of the patient 104, or the like. In some examples, the airway pressure sensor includes multiple ducts and/or multiple sensors on either side of a resistive, pressure-differential-producing element.

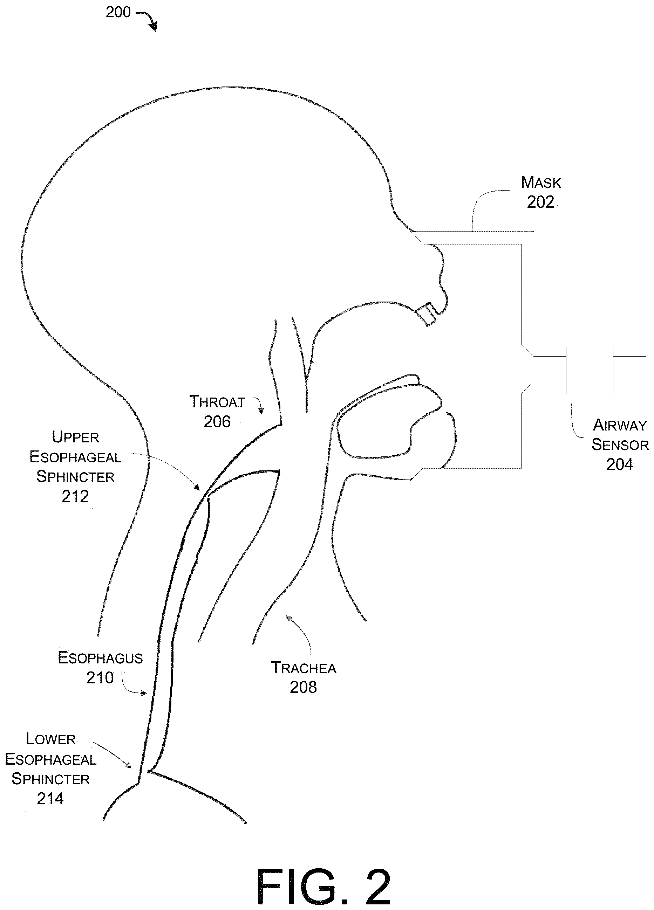

FIG. 2 illustrates an example patient 200 receiving assisted ventilation via a mask 202. The mask 202, in some examples, is in turn connected to a ventilation device, such as a mechanical ventilator or a bag that is manually squeezed by a rescuer. In various cases, an airway sensor 204 is integrated with the mask 202. For example, the airway sensor 204 may be disposed in a duct between the mask 202 and a source of pressure, such as the mechanical ventilator or bag. The airway sensor 204, in various implementations, may detect one or more airway parameters.

As shown, the mask 202 is disposed on a face of the patient 200. In particular cases, a seal is formed between the skin of the face of the patient 200 and the mask 202. When a pressure induced in the interior of the mask 202 (e.g., by the mechanical ventilator or bag) is greater than the pressure in the airway of the patient 200, air flows into the airway of the patient 200. In addition, when the pressure in the interior mask 202 is lowered such that it is less than the pressure in the airway of the patient 200, air flows form the airway of the patient 200 into the mask 202. Accordingly, the mask 202 may assist ventilation of the patient 200 when the patient 200 is unable to sufficiently breathe on their own.

In various implementations, the airway of the patient 200 includes the throat 206 and the trachea 208 of the patient 200. Although not illustrated in FIG. 2, the trachea 208 may be further connected to the lungs of the patient 200. The lungs may be responsible for oxygenating blood of the patient 200 and removing CO₂ from the blood of the patient 200. Based on the delivery of air through the trachea 208 and to the lungs of the patient 200, the oxygen from the delivered air can be used to oxygenate the blood. Further, based on the removal of air from the lungs through the trachea 208, the CO₂ from the blood can be removed. Thus, inducing bidirectional airflow through the trachea 208 can provide assisted ventilation to the patient 200.

The throat 206 of the patient 200, however, is also connected to an esophagus 210. The esophagus 210 connects the throat 206 to the stomach (not illustrated) of the patient 200. In particular, the esophagus 210 includes an upper esophageal sphincter 212, which is connected to the throat 206, and a lower esophageal sphincter 214, which is connected to the stomach.

If greater than a threshold pressure is induced in the throat 206 (e.g., due to the pressure induced through the mask 202), the upper esophageal sphincter 212 and/or lower esophageal sphincter 214 are forced open. As a result, air can flow into the stomach and the stomach can become insufflated with air. Once the stomach has become insufflated with air, gastric contents in the stomach can be regurgitated from the stomach, to the esophagus 210, and into the throat 206 of the patient 200. These contents, in some cases, can travel through the trachea 208 and into the lungs of the patient 200. In various cases, the gastric contents interfere with the delivery of air to and from the lungs of the patient 200. In some cases, the gastric contents are sufficiently acidic such that they can cause damage to the throat 206, the trachea 208, and the lungs of the patient 200. Thus, it is advantageous to prevent the mask 202 from imposing greater than the threshold pressure to the airway of the patient 200.

In various implementations, the pressure in the mask 202 is monitored via the airway sensor 204. For example, the airway sensor 204 may transmit data indicative of the pressure in the mask 202 to a device, such as a monitor (e.g., the monitor 106 described above with reference to FIG. 1) or another device. In various cases, the airway sensor 204 and the device are communicatively coupled by one or more wired interfaces, one or more wireless interfaces, or a combination thereof. In some cases, the device outputs the pressure. In various examples, the device compares the pressure in the mask 202 to a threshold pressure, which may represent a pressure lower than the minimum pressure that opens the upper esophageal sphincter and/or the lower esophageal sphincter. When the device determines that the pressure in the mask 202 is above the threshold pressure, the device may output an alert or alarm to a user. Accordingly, the user may adjust assisted ventilation parameters of the patient 200 in order to prevent the patient 200 from regurgitating their gastric contents. In some cases, the device may determine a metric based on a fraction of time within a time window at which the pressure in the mask 202 exceeds the threshold pressure, and may output the metric to the user or may alarm when the metric exceeds a threshold.

In some cases, the device infers the pressure in the airway of the patient 200 based on the pressure in the mask 202. The device may use the pressure detected by the airway sensor 202 to determine one or more metrics. In various cases, the device outputs the metric(s) and/or alarms based on comparing the metric(s) to one or more thresholds.

According to some implementations, the airway sensor 204 includes a flow sensor that is configured to detect a flow rate of air through the mask 202. In some cases, the flow sensor is a bidirectional flow sensor, such that it can measure a flow rate of air flowing from the mask to the ventilation device and from the ventilation device to the mask. The airway sensor 202 may transmit data indicative of the flow rate in the mask 202 to the device. The device, in some cases, outputs the flow rate. In some examples, the device compares the flow rate to a threshold and alerts if the flow rate exceeds a threshold flow rate. In some implementations, the device determines a metric based on the flow rate. The device, in various examples, outputs the metric and/or alarms based on comparing the metric to a threshold.

In various cases, the airway sensor 204 includes a CO₂ sensor configured to detect a CO₂ level (e.g., a partial pressure of) in the mask 202. The airway sensor 204 may transmit data indicative of the CO₂ level to the device, which may output the CO₂ level and/or analyze the CO₂ level. For example, the device may alarm based on comparing the CO₂ to a threshold. In some cases, the device generates a metric based on the CO₂ and outputs the metric and/or alarms based on comparing the metric to a threshold.

According to various implementations, the device detects a leak between the mask 202 and the face of the patient 200 based on one or more of the parameters detected by the airway sensor 204. For example, the device detects the leak when the volume of air delivered from the ventilation device into the mask during insufflation is greater than the volume of air delivered from the mask 202 to the ventilation device during expiration. The device, for example, determines the volume of air flowing through the mask 202 based on the flow rate detected by the airway sensor 204. In some implementations, the device alarms based on the detected leak. For instance, the device instructs the user to check for the leak, to use two hands to hold down the mask 202 on the face of the patient 200, or the like.

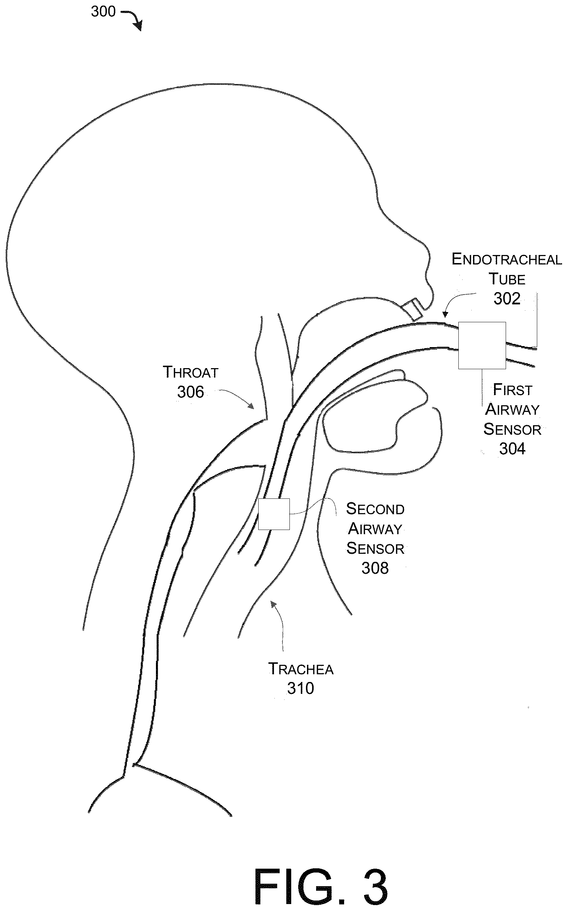

FIG. 3 illustrates an example intubated patient 300 receiving assisted ventilation via an endotracheal tube 302. The endotracheal tube 302, in some examples, is in turn connected to a ventilation device, such as a mechanical ventilator or a bag that is manually squeezed by a rescuer. In various cases, a first airway sensor 304 and a second airway sensor 306 are integrated with the endotracheal tube 302. For example, the first airway sensor 304 may be disposed in the endotracheal tube 302 between a throat 306 and the ventilation device. The second airway sensor 308 may be disposed in the endotracheal tube 302 between the throat 306 and the lungs of the patient 300. In some cases, the second airway sensor 308 is disposed at a different position, but is fluidically coupled to a region between the throat 306 and the lungs of the patient 300. For instance, the second airway sensor 308 is disposed out of the airway of the patient 300, but is connected to a tube that includes an open end at the distal end of the endotracheal tube 302. The first airway sensor 304 and/or the second airway sensor 308, in various implementations, may detect one or more airway parameters.

During intubation, the patient 300 may be administered a sedative and a paralytic. Once the patient 300 is paralyzed by the paralytic, the endotracheal tube 302 may be inserted through the throat 306 and into a trachea 308 of the patient 300. The trachea 308 is connected to the lungs of the patient 300. In various implementations, the endotracheal tube is connected to a ventilation device that pushes air into the lungs through the endotracheal tube 302 and allows air from the lungs to exit through the endotracheal tube 302.

In various cases, excessive pressure in the endotracheal tube 302 can cause damage to the lungs of the patient 300. To avoid problems associated with excessive airway pressures, in some implementations, the first airway sensor 304 and/or the second airway sensor 308 may detect a pressure in the endotracheal tube 302. The first airway sensor 304 and/or the second airway sensor 308 may transmit data indicative of the pressure to a device (e.g., the monitor 106 described above with reference to FIG. 1). For instance, the first airway sensor 304 and/or the second airway sensor 308 are connected to the device via one or more wired interfaces, one or more wireless interfaces, or a combination thereof. In some cases, the device outputs the pressure. In some implementations, the device analyzes the pressure. For example, the device compares the detected pressure to a threshold pressure, which may be less than or equal to a minimum pressure associated with damage to the lungs of the patient 300. If the device determines that the detected pressure exceeds the threshold pressure, the device may output an alarm. In some cases, the device calculates a metric based on the pressure. For example, the device may determine the metric based on a fraction of time in a time interval during which the detected pressure exceeded the threshold pressure. Accordingly, a user (e.g., a rescuer) may use the feedback from the monitor 106 to avoid subjecting the airway of the patient 300 to excess pressures.

In various cases, an airway device with an inflatable cuff or mask, such as a cuffed endotracheal tube, or an SGA with an inflatable cuff or laryngeal mask, may be inserted in the patient 300. The volume of the inflated cuff or mask may be susceptible to change if the patient 300 is transported in an aircraft or other vehicle that may significantly change altitude, such that an ambient pressure may increase or decrease while the patient 300 has an airway device deployed in their airway. Although not illustrated in FIG. 3, in some cases, an inflatable cuff is disposed between the endotracheal tube 302 and the trachea 308. When the volume of air in the inflatable cuff is reduced, due to a decrease in altitude and consequent increase in ambient pressure, for example, the endotracheal tube 302 can become unsealed from the airway of the patient 300, which can interfere with the administration of assisted ventilation. When the volume of air in the inflatable cuff is increased, due to an increase in altitude, for example, the cuff of the endotracheal tube 302 can exert increased pressure on the surrounding tissue, causing damage.

In various examples, the first airway sensor 304 and/or the second airway sensor 308 detects a flow rate of air flowing through the endotracheal tube 302. For example, the first airway sensor 304 and/or the second airway sensor 308 includes a bidirectional flow sensor. The first airway sensor 304 and/or the second airway sensor 308, in various cases, transmits data indicative of the flow rate to the device. In some cases, the device outputs the flow rate. In some implementations, the device analyzes the flow rate. For example, the device compares the flow rate to a threshold, and alarms if the flow rate exceeds the threshold. In some instances, the device determines a metric based on the flow rate and outputs the metric and/or alarms based on the metric. Accordingly, the user may use the feedback from the monitor 106 to avoid subjecting the airway of the patient 300 to excessive flow rates.

In some instances, the first airway sensor 304 and/or the second airway sensor 308 may detect CO₂ level (e.g., a partial pressure of CO₂) in the airway of the patient 300. The first airway sensor 304 and/or the second airway sensor 308 may transmit data indicative of the CO₂ level to the device. In some cases, the device outputs the CO₂ level. In some implementations, the device alarms if the CO₂ level exceeds a first threshold or is lower than a second threshold. In various cases, the device generates a metric based on the CO₂ level. According to some implementations, the device outputs the metric and/or alarms based on the metric.

In particular cases, the sedative and paralytic used to intubate the patient 300 begins to wear off. If the endotracheal tube 302 remains in the airway of the patient 300 when the patient is no longer sedated, the patient 300 may experience extreme discomfort and damage. To avoid this problem in various cases, the device detects the presence of spontaneous breaths by the patient 300 based on one or more of the parameters detected by the first airway sensor 304 and/or the second airway sensor 306. In various cases, the device alarms based on detecting one or more spontaneous breaths. The presence of spontaneous breaths indicates that the paralytic is wearing off. In some cases, the paralytic wears off substantially simultaneously as the sedative wears off. Accordingly, the user may rely on the alarm to identify when to redose the patient with the sedative and/or paralytic.

Although not illustrated in FIG. 3, in some cases, the endotracheal tube 302 can be substituted for an SGA, which includes a duct inserted across the glottis, which is disposed between the throat 306 and trachea 308 of the patient 300. The SGA is not inserted inside of the trachea 308, for example.

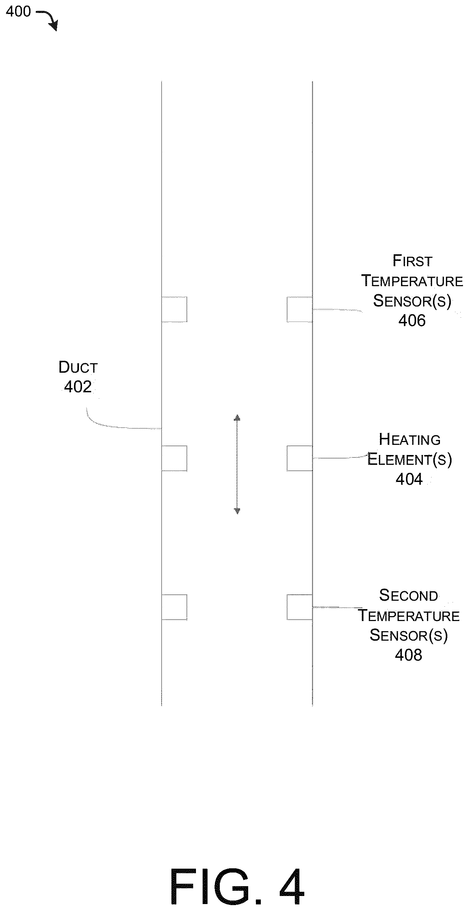

FIG. 4 illustrates an example of a bidirectional airflow sensor 400. In various cases, the bidirectional airflow sensor 400 is integrated with, or disposed inside of, a duct 402. The duct, for instance, is a tube, a pipe, or other element through which airflow can pass bidirectionally.

The bidirectional airflow sensor 400 includes one or more heating elements 404, one or more first temperature sensors 406, and one or more second temperature sensors 408, all of which are disposed inside of and/or integrated with the duct 402. For example, the heating element(s) 404, the first temperature sensor(s) 406, and the second temperature sensor(s) 408 are disposed at a sidewall of the duct 402. Although not illustrated in FIG. 4, in some implementations, the heating element(s) 404, the first temperature sensor(s) 406, and the second temperature sensor(s) 408 are positioned such that they do not substantially impact the resistance to airflow within the duct 402. The first temperature sensor(s) 406 and the second temperature sensor(s) 408 include thermocouples, thermistors, or the like, in various cases.

The heating element(s) 404 are configured to generate heat in the duct 402. As air flows through the duct 402 in a first direction, the heated air is carried to the first temperature sensor(s) 406. The first temperature sensor(s) 406 are configured to detect the temperature of the air flowing through the duct 402. The temperature is based on the temperature of the air originally heated by the heating element(s) 404 and the flow rate of the air flowing from the heating element(s) 404 to the first temperature sensor(s) 406. Accordingly, if the heat output by the heating element(s) 404 is known, the flow rate of the air flowing from the heating element(s) 404 to the first temperature sensor(s) 406 can be derived based on the temperature detected by the first temperature sensor(s) 406. In some examples, the temperature detected by the second temperature sensor(s) 408 is further used to distinguish between changes caused by the temperature of the air entering the duct 402 and the contribution of heat from the heating element(s) 404, thereby enhancing the accuracy of the detected flow rate.

Similarly, the bidirectional airflow sensor determines the flow rate of air flowing through the duct 402 in a second direction that is opposite to the first direction. As the air flows through the duct 402 in the second direction, the heated air is carried to the second temperature sensor(s) 408. The second temperature sensor(s) 408 are configured to detect the temperature of the air flowing through the duct 402. Accordingly, the flow rate in the second direction can be derived based on the temperature detected by the second temperature sensor(s) 408. In some examples, the temperature detected by the first temperature sensor(s) 406 is further used to distinguish between changes caused by the temperature of the air entering the duct 402 and the contribution of heat from the heating element(s) 404, thereby enhancing the accuracy of the detected flow rate

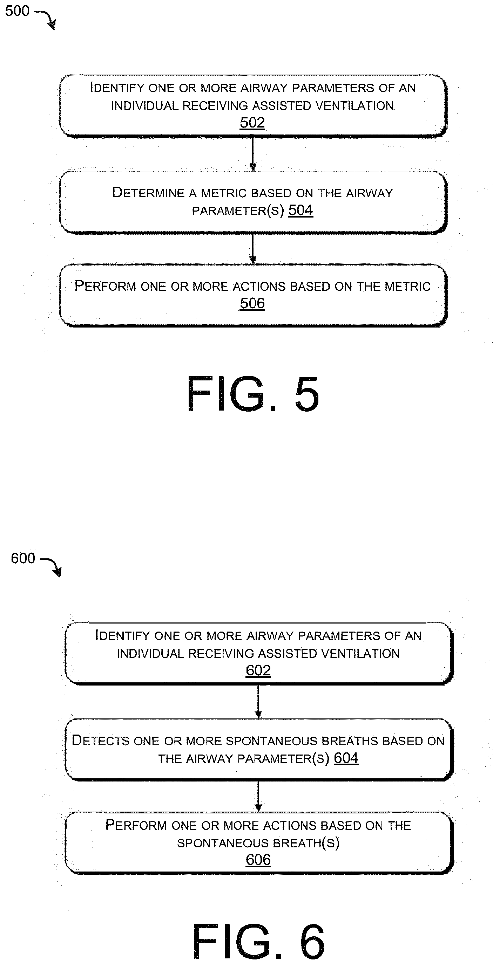

FIG. 5 illustrates an example process 500 for analyzing the airway of an individual. The process 500 can be performed by an entity, such as the monitor 106 described above with reference to FIG. 1 or a computing device.

At 502, the entity identifies one or more airway parameters of an individual (e.g., a patient) receiving assisted ventilation. In some implementations, the entity receives, from one or more airway sensor(s), a signal indicative of the airway parameter(s). For example, the signal is received over a wired interface, a wireless interface, or a combination thereof. In some cases, the airway sensor(s) are integrated into the entity. Accordingly, in some cases, the entity detects the airway parameter(s) at 502. The airway parameter(s) include, for instance, airway pressure (e.g., positive end-expiratory pressure (PEEP), plateau pressure, peak inspiratory pressure (PIP), etc.), airway flow (e.g., flow rate), airway volume (e.g., inspiratory volume, expiratory volume, tidal volume, or minute volume), respiration/ventilation rate, inspiratory and/or expiratory time, partial pressure of CO₂, end tidal CO₂, partial pressure of O₂, end tidal O2, and so on. In some cases, the airway sensor(s) are integrated with a ventilation device, such as a bag-valve mask, an intubation tube, supraglottic airway device, or the like.

At 504, the entity determines a metric based on the airway parameter(s). The metric is indicative of a physiological condition of the individual and/or a quality of assisted ventilation administered to the individual.

For example, the entity determines a positive pressure fraction index based on the detected pressure in the airway over a time interval. In various implementations, the positive pressure fraction index is determined based on a percentage and/or fraction of the time interval at which the detected pressure exceeds a particular threshold. In some cases, the threshold is selected based on the ventilation device and/or is input into the entity by a user.

In some implementations, the entity determines a spontaneous breathing index based on the airway parameter(s). The spontaneous breathing index is based on a characteristic (such as the amount) of PPV breaths, a characteristic (such as the amount) of spontaneous breaths, or both, experienced by the individual. For example, the spontaneous breathing index is a quotient of the number and/or rate of PPV breaths versus the number and/or rate of spontaneous breaths. In various cases, the entity distinguishes between PPV breaths and spontaneous breaths based on a CO₂ level in the airway of the individual over time and/or a shape of a waveform representing the CO₂ level over time. In some examples, the entity distinguishes the PPV breaths from the spontaneous breaths based solely on the CO₂ waveform. In some cases, the entity uses another airway parameter to identify the different types of breaths. For example, the entity distinguishes PPV breaths and spontaneous breaths based on an airway flow rate waveform and/or a derived tidal volume of the individual. In some cases, spontaneous breaths (at least in individuals receiving PPV in emergency care situations) have a different flow envelope and/or tidal volume than PPV breaths, which can be used by the entity to distinguish the types of breaths.

According to various examples, the entity determines a variability index based on the airway parameter(s). In various implementations, the entity detects multiple breath events (e.g., where an individual breath event is an inspiratory event followed by an expiratory event, or vice versa) of the individual. The entity determines tidal volumes associated with the individual breath events based on the detected flow rates over time. In various cases, the entity determines a tidal volume variability by determining a mathematical variance of the tidal volumes of the individual breath events. In some cases, the tidal volume variability is associated with the asynchrony of the individual.

Other metrics are also possible. In some cases, the entity determines a PEEP and/or PIP of the individual based on the airway parameter(s). In some other cases, the entity determines a minute ventilation, a CO₂ extraction rate, an airway leak fraction, a positive pressure fraction, a spontaneous breathing index and/or variability index (e.g. a tidal volume variability index or a rate variability index). According to some examples, the entity determines a hypoxemia dose index based on the airway parameter(s). In some implementations, the entity determines a hyperventilation index based on the airway parameter(s).

At 506, the entity performs one or more actions based on the metric. According to various implementations, the entity outputs the metric. The entity, in some implementations, stores data indicative of the metric and/or transmits data indicative of the metric (e.g., in a data stream) to an external device. In some cases, the entity compares the metric to a first threshold and/or a second threshold. The entity, for instance, alarms if the metric is greater than the first threshold and/or lower than the second threshold. In some cases, the entity outputs an instruction to a user based on the metric. For example, the entity outputs an instruction to administer anesthesia to the individual, to check for and/or address a problem (e.g., a leak) associated the ventilation device, or to change ventilation parameters of the individual. In some implementations, the entity controls a mechanical ventilator based on the metric. For example, the entity may cause the mechanical ventilator to change the ventilation parameters of the individual. Examples of such changes are described in Chapman *et al.,* ANNALS OF BIOMEDICAL ENGINEERING. 1985; 13:359 372, which is incorporated by reference herein. In some cases, the entity triggers detection of another parameter of the individual based on the metric. For instance, the entity causes a blood pressure sensor (e.g., a blood pressure cuff) to measure a blood pressure of the individual in response to determining that the positive pressure fraction exceeds a threshold, or in response to a change in heart rate that exceeds a threshold percentage and/or crosses a threshold value.

FIG. 6 illustrates an example process 600 for evaluating spontaneous breathing of a individual during PPV. The process 600 can be performed by an entity, such as the monitor 106 described above with reference to FIG. 1 or a computing device.

At 602, the entity identifies one or more airway parameters of an individual receiving assisted ventilation. In some implementations, the entity receives, from one or more airway sensor(s), a signal indicative of the airway parameter(s). For example, the signal is received over a wired interface, a wireless interface, or a combination thereof. In some cases, the airway sensor(s) are integrated into the entity. Accordingly, in some cases, the entity detects the airway parameter(s) at 602. The airway parameter(s) include, for instance, airway pressure (e.g., positive end-expiratory pressure (PEEP), plateau pressure, peak inspiratory pressure (PIP), etc.), airway flow (e.g., flow rate), airway volume (e.g., inspiratory volume, expiratory volume, tidal volume, or minute volume), ventilation rate, inspiratory and/or expiratory time, partial pressure of CO₂, end tidal CO₂, partial pressure of O₂, end tidal O₂ and so on. In some cases, the airway sensor(s) are integrated with a ventilation device, such as a bag-valve mask, an intubation tube, supraglottic airway device, or the like.

At 604, the entity detects one or more spontaneous breaths based on the airway parameter(s). In various cases, the entity distinguishes between PPV breaths and spontaneous breaths based on a CO₂ level in the airway of the individual over time. In some examples, the entity distinguishes the PPV breaths from the spontaneous breaths based solely on the CO₂ waveform. In some cases, the entity uses another airway parameter to identify the different types of breaths. For example, the entity distinguishes PPV breaths and spontaneous breaths based on an airway flow rate waveform and/or a derived tidal volume of the individual. In some cases, spontaneous breaths (at least in individuals receiving PPV in emergency care situations) have a different flow envelope and/or tidal volume than PPV breaths, which can be used by the entity to distinguish the types of breaths. In some examples, the entity distinguishes PPV breaths and spontaneous breaths based on the airway pressure of the individual. In various cases, the entity distinguishes PPV breaths and spontaneous breaths based on a combination of two or more airway parameters of the individual, such as an assessment of the presence or absence of a rise and fall in positive airway pressure temporally corresponding to an inspiratory phase of a CO₂ waveform.

At 606, the entity performs one or more actions based on the spontaneous breath(s). According to some implementations, the entity outputs an indication of the spontaneous breath(s) and/or the PPV breaths. The entity, in some examples, transmits data indicative of the spontaneous breath(s) and/or the PPV breaths (e.g., in a data stream) to an external device and/or stores data indicative of the spontaneous breath(s). The stored data can be used for post-event review.

In some cases, the entity removes a component due to the spontaneous breaths from an airway parameter waveform, and analyzes the resultant waveform. For example, the resultant waveform is representative of PPV breaths administered to the individual. In some cases, the entity derives a parameter and/or metric based on the resultant waveform, and outputs the parameter and/or metric. Thus, a user can adjust a condition of assisted ventilation provided to the individual based on the parameter and/or metric.

In some cases, the entity compares the number and/or rate of spontaneous breaths to a first threshold and/or a second threshold. The entity, for instance, alarms if the metric is greater than the first threshold (which may indicate that the individual is regaining consciousness or a paralytic is wearing off) and/or lower than the second threshold (which may indicate that the individual is in need of assisted ventilation and can no longer breathe independently). In some cases, the entity outputs an instruction to a user based on the metric. For example, the entity outputs an instruction to administer anesthesia to the individual or to administer assisted ventilation to the individual. In some implementations, the entity controls a mechanical ventilator based on the metric. For example, the entity may cause the mechanical ventilator to activate when the number or rate of spontaneous breaths is below the second threshold.

In some cases, the entity creates a time-stamped event in a data record, for example if the metric is lower than the second threshold (which may indicate the time of onset of paralysis from an administered paralytic medication). The entity stores the data record and/or transmits a signal indicative of the data record to an external device. In various simplementations, the entity identifies a transition from a time interval with some spontaneous breaths (e.g. intermingled with PPV breaths) to a subsequent time interval with no spontaneous breaths. Optionally the entity identifies additional contextual information, such as the type of ventilation device in place and/or the type of procedure being performed (e.g. RSI) on the individual. The additional contextual information is also stored in the data record, for example. The time of the transition corresponds to the time of paralytic onset, which allows the entity to automatically (e.g., without any other user input) identify 1) that a paralytic was given, and 2) the time that it was administered (which would be a short and predictable interval prior to the onset of action).

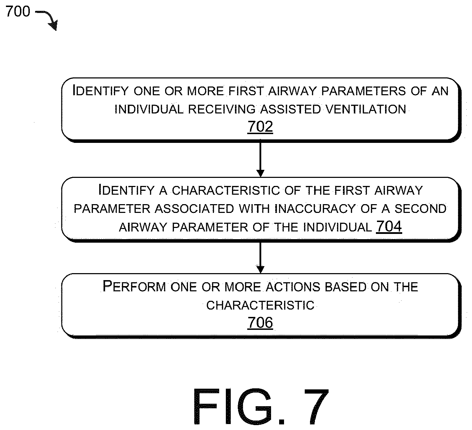

FIG. 7 illustrates an example process 700 for evaluating the accuracy of an airway parameter. The process 700 can be performed by an entity, such as the monitor 106 described above with reference to FIG. 1 or a computing device.

At 702, the entity identifies one or more first airway parameters of an individual receiving assisted ventilation. In some implementations, the entity receives, from one or more airway sensor(s), a signal indicative of the airway parameter(s). For example, the signal is received over a wired interface, a wireless interface, or a combination thereof. In some cases, the airway sensor(s) are integrated into the entity. Accordingly, in some cases, the entity detects the airway parameter(s) at 602. The airway parameter(s) include, for instance, airway pressure (e.g., positive end-expiratory pressure (PEEP), plateau pressure, peak inspiratory pressure (PIP), etc.), airway flow (e.g., flow rate), airway volume (e.g., inspiratory volume, expiratory volume, tidal volume, or minute volume), ventilation rate, inspiratory and/or expiratory time, partial pressure of CO₂, end tidal CO₂, partial pressure of O₂, end tidal O₂, and so on. In some cases, the airway sensor(s) are integrated with a ventilation device, such as a bag-valve mask, an intubation tube, supraglottic airway device, or the like.

At 704, the entity detects a characteristic of the first airway parameter(s) associated with inaccuracy of a second airway parameter of the individual. In particular examples, the entity analyzes a CO₂ level of the individual over time. The detected characteristic, for example, is an absence of a plateau phase in the waveform of the CO₂. In some cases, the entity detects the characteristic based on comparing a spectral signature of the CO₂ waveform to a spectral signature of a sample CO₂ waveform that includes a plateau phase. In some examples, the entity detects the characteristic upon determining that a slope at which the CO₂ waveform descends from a peak CO₂ level is not sufficiently steep (e.g., if the slope is greater than a particular threshold). In various cases, the entity detects the characteristic based on determining that a time interval that begins at the maximum CO₂ level and that ends at the minimum CO₂ level is greater than a threshold time interval. The characteristic of the CO₂ level may be indicative of a leak in a fluidic circuit connecting the airway of the individual with the ventilation device. Accordingly, this characteristic is associated with inaccuracy in an EtCO₂ of the individual that is derived based on the CO₂ level and/or a tidal volume of the individual detected based on a flow sensor in the fluidic circuit.

At 706, the entity performs one or more actions based on the characteristic of the first airway parameter(s). In some cases, the entity outputs an indication of the characteristic. According to some implementations, the entity stores data indicating the characteristic and/or transmits data indicating the characteristic to an external device (e.g., in a data stream).

In some cases, the entity alarms based on the characteristic. For example, the entity outputs a warning indicating that the second airway parameter (e.g., EtCO₂, flow rate, tidal volume, etc.) is inaccurate, outputs an instruction to check for leaks between the individual and the ventilation device, outputs an instruction to hold down a mask of the ventilation device on the face of the individual with both hands (e.g., to prevent leaks), outputs an instruction to use another parameter (e.g., SpO₂) to confirm a condition of the individual, or the like.

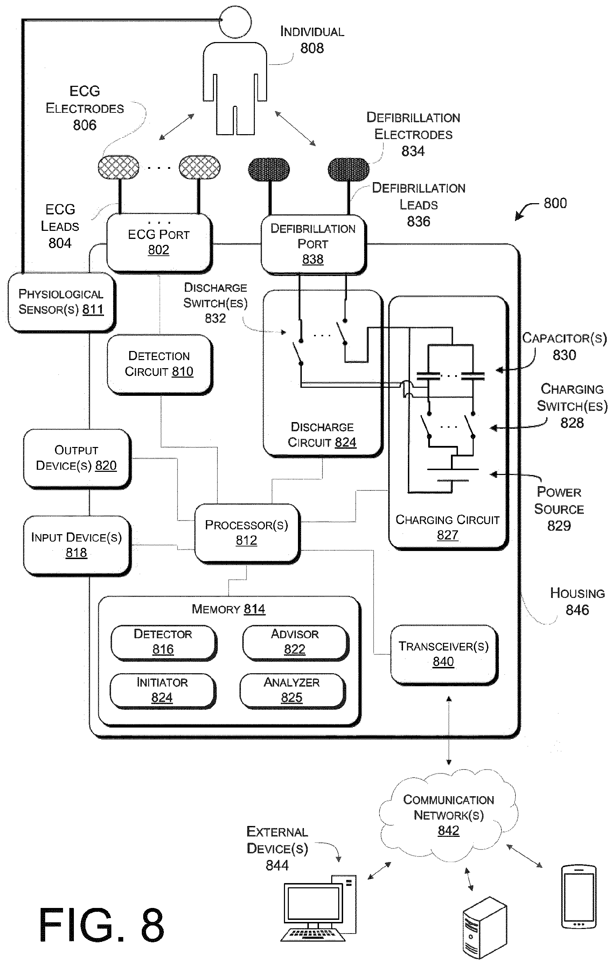

FIG. 8 illustrates an example of an external defibrillator 800 configured to perform various functions described herein. For example, the external defibrillator 800 is the monitor 106 described above with reference to FIG. 1.

The external defibrillator 800 includes an electrocardiogram (ECG) port 802 connected to multiple ECG leads 804. In some cases, the ECG leads 804 are removeable from the ECG port 802. For instance, the ECG leads 804 are plugged into the ECG port 802. The ECG leads 804 are connected to ECG electrodes 806, respectively. In various implementations, the ECG electrodes 806 are disposed on different locations on an individual 808. A detection circuit 810 is configured to detect relative voltages between the ECG electrodes 806. These voltages are indicative of the electrical activity of the heart of the individual 808. In various cases, the individual 808 is receiving assisted ventilation from a ventilation device.

In various implementations, the ECG electrodes 806 are in contact with the different locations on the skin of the individual 808. In some examples, a first one of the ECG electrodes 806 is placed on the skin between the heart and right arm of the individual 808, a second one of the ECG electrodes 806 is placed on the skin between the heart and left arm of the individual 808, and a third one of the ECG electrodes 806 is placed on the skin between the heart and a leg (either the left leg or the right leg) of the individual 808. In these examples, the detection circuit 808 is configured to measure the relative voltages between the first, second, and third ECG electrodes 806. Respective pairings of the ECG electrodes 806 are referred to as "leads," and the voltages between the pairs of ECG electrodes 806 are known as "lead voltages." In some examples, more than three ECG electrodes 806 are included, such that 5-lead or 12-lead ECG signals are detected by the detection circuit 810.

The detection circuit 810 includes at least one analog circuit, at least one digital circuit, or a combination thereof. The detection circuit 810 receives the analog electrical signals from the ECG electrodes 806, via the ECG port 802 and the ECG leads 804. In some cases, the detection circuit 810 includes one or more analog filters configured to filter noise and/or artifact from the electrical signals. The detection circuit 810 includes an analog-to-digital (ADC) in various examples. The detection circuit 810 generates a digital signal indicative of the analog electrical signals from the ECG electrodes 806. This digital signal can be referred to as an "ECG signal" or an "ECG."

In some cases, the detection circuit 810 further detects an electrical impedance between at least one pair of the ECG electrodes 806. For example, the detection circuit 810 includes, or otherwise controls, a power source that applies a known voltage across a pair of the ECG electrodes 806 and detects a resultant current between the pair of the ECG electrodes 806 (or applies a known current and detects a resultant voltage between the pair of ECG electrodes 806). The impedance is generated based on the applied voltage and the resultant current. In various cases, the impedance corresponds to respiration of the individual 808, chest compressions performed on the individual 808, pulsatile cardiac output of the individual, and other physiological states of the individual 808. In various examples, the detection circuit 810 includes one or more analog filters configured to filter noise and/or artifact from the resultant current. The detection circuit 810 generates a digital signal indicative of the impedance using an ADC. This digital signal can be referred to as an "impedance signal" or an "impedance."

In some cases, the detection circuit 810 receives, from one or more other physiological sensors 811, analog signals indicative of one or more parameters of the individual 808. The physiological sensor(s) 811, for instance, include one or more airway sensors, a blood pressure cuff configured to detect a blood pressure of the individual 808, an oximetry sensor configured to detect an SpO₂ and/or rSO₂ of the individual 808, or the like. Examples of airway sensors are described above with reference to FIGS. 1-3.

The detection circuit 810 provides the parameter signal(s) to one or more processors 812 in the external defibrillator 800. In some implementations, the processor(s) 812 includes a central processing unit (CPU), a graphics processing unit (GPU), both CPU and GPU, or other processing unit or component known in the art.

The processor(s) 812 is operably connected to memory 814. In various implementations, the memory 812 is volatile (such as random access memory (RAM)), nonvolatile (such as read only memory (ROM), flash memory, etc.) or some combination of the two. The memory 814 stores instructions that, when executed by the processor(s) 812, causes the processor(s) 812 to perform various operations. In various examples, the memory 814 stores methods, threads, processes, applications, objects, modules, any other sort of executable instruction, or a combination thereof. In some cases, the memory 814 stores files, databases, or a combination thereof. In some examples, the memory 814 includes, but is not limited to, RAM, ROM, electrically erasable programmable read-only memory (EEPROM), flash memory, or any other memory technology. In some examples, the memory 814 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 812 and/or the external defibrillator 800. In some cases, the memory 814 at least temporarily stores the ECG signal and/or the impedance signal.

In various examples, the memory 814 includes a detector 816, which causes the processor(s) 812 to determine, based on the ECG signal and/or the impedance signal, whether the individual 808 is exhibiting a particular heart rhythm. For instance, the processor(s) 812 determines whether the individual 808 is experiencing a shockable rhythm that is treatable by defibrillation. Examples of shockable rhythms include ventricular fibrillation (VF) and ventricular tachycardia (V-Tach). In some examples, the processor(s) 812 determines whether any of a variety of different rhythms (e.g., asystole, sinus rhythm, atrial fibrillation (AF), etc.) are present in the ECG signal.

The processor(s) 812 is operably connected to one or more input devices 818 and one or more output devices 820. Collectively, the input device(s) 818 and the output device(s) 820 function as an interface between a user and the defibrillator 800. The input device(s) 818 is configured to receive an input from a user and includes at least one of a keypad, a cursor control, a touch-sensitive display, a voice input device (e.g., a speaker), a haptic feedback device, or any combination thereof. The output device(s) 820 includes at least one of a display, a speaker, a haptic output device, a printer, or any combination thereof. In various examples, the processor(s) 812 causes a display among the input device(s) 818 to visually output a waveform of the ECG signal and/or the impedance signal. In some implementations, the input device(s) 818 includes one or more touch sensors, the output device(s) 820 includes a display screen, and the touch sensor(s) are integrated with the display screen. Thus, in some cases, the external defibrillator 800 includes a touchscreen configured to receive user input signal(s) and visually output physiological parameters and/or metrics. In some implementations, the output device(s) 820 output warnings and/or instructions to a user.

In some examples, the memory 814 includes an advisor 822, which, when executed by the processor(s) 812, causes the processor(s) 812 to generate advice and/or control the output device(s) 820 to output the advice to a user (e.g., a rescuer). In some examples, the processor(s) 812 provides, or causes the output device(s) 820 to provide, an instruction to perform CPR on the individual 808. In some cases, the processor(s) 812 evaluates, based on the ECG signal, the impedance signal, or other physiological parameters, CPR being performed on the individual 808 and causes the output device(s) 820 to provide feedback about the CPR in the instruction. According to some examples, the processor(s) 812, upon identifying that a shockable rhythm is present in the ECG signal, causes the output device(s) 820 to output an instruction and/or recommendation to administer a defibrillation shock to the individual 808.

The memory 814 also includes an initiator 824 which, when executed by the processor(s) 812, causes the processor(s) 812 to control other elements of the external defibrillator 800 in order to administer a defibrillation shock to the individual 808. In some examples, the processor(s) 812 executing the initiator 824 selectively causes the administration of the defibrillation shock based on determining that the individual 808 is exhibiting the shockable rhythm and/or based on an input from a user (received, e.g., by the input device(s) 818. In some cases, the processor(s) 812 causes the defibrillation shock to be output at a particular time, which is determined by the processor(s) 812 based on the ECG signal and/or the impedance signal.

In various implementations, the memory 814 further includes an analyzer 825, which, when executed by the processor(s) 812, causes the processor(s) 812 to analyze one or more parameters of the individual 808. In various examples, the analyzer 825 is configured to analyze one or more airway parameters of the individual 808 and to perform one or more actions based on the airway parameter(s). For instance, the analyzer 825 causes the processor(s) 812 to identify one or more airway parameters of the individual 808, determine a metric based on the airway parameter(s), and perform one or more actions based on the metric. In some cases, the analyzer 825 causes the processor(s) 812 to detect one or more spontaneous breaths based on the airway parameter(s) and perform one or more actions based on the spontaneous breath(s). According to various implementations, the analyzer 825 causes the processor(s) 812 to identify a characteristic of a first airway parameter associated with inaccuracy of a second airway parameter, and to perform one or more actions based on the characteristic.

The processor(s) 812 is operably connected to a charging circuit 827 and a discharge circuit 824. In various implementations, the charging circuit 827 includes a power source 829, one or more charging switches 828, and one or more capacitors 830. The power source 829 includes, for instance, a battery. The processor(s) 812 initiates a defibrillation shock by causing the power source 829 to charge at least one capacitor among the capacitor(s) 830. For example, the processor(s) 812 activates at least one of the charging switch(es) 828 in the charging circuit 827 to complete a first circuit connecting the power source 829 and the capacitor to be charged. Then, the processor(s) 812 causes the discharge circuit 824 to discharge energy stored in the charged capacitor across a pair of defibrillation electrodes 830, which are in contact with the individual 808. For example, the processor(s) 812 deactivates the charging switch(es) 828 completing the first circuit between the capacitor(s) 830 and the power source 829, and activates one or more discharge switches 832 completing a second circuit connecting the charged capacitor 830 and at least a portion of the individual 808 disposed between defibrillation electrodes 834.

The energy is discharged from the defibrillation electrodes 834 in the form of a defibrillation shock. For example, the defibrillation electrodes 834 are connected to the skin of the individual 808 and located at positions on different sides of the heart of the individual 808, such that the defibrillation shock is applied across the heart of the individual 808. The defibrillation shock, in various examples, depolarizes a significant number of heart cells in a short amount of time. The defibrillation shock, for example, interrupts the propagation of the shockable rhythm (e.g., VF or V-Tach) through the heart. In some examples, the defibrillation shock is 200 J or greater with a duration of about 0.015 seconds. In some cases, the defibrillation shock has a multiphasic (e.g., biphasic) waveform. The discharge switch(es) 832 are controlled by the processor(s) 812, for example. In various implementations, the defibrillation electrodes 834 are connected to defibrillation leads 836. The defibrillation leads 836 are connected to a defibrillation port 838, in implementations. According to various examples, the defibrillation leads 836 are removable from the defibrillation port 838. For example, the defibrillation leads 836 are plugged into the defibrillation port 838.

In various implementations, the processor(s) 812 is operably connected to one or more transceivers 840 that transmit and/or receive data over one or more communication networks 842. For example, the transceiver(s) 840 includes a network interface card (NIC), a network adapter, a local area network (LAN) adapter, or a physical, virtual, or logical address to connect to the various external devices and/or systems. In various examples, the transceiver(s) 840 includes any sort of wireless transceivers capable of engaging in wireless communication (e.g., radio frequency (RF) communication). For example, the communication network(s) 842 includes one or more wireless networks that include a 3^{rd} Generation Partnership Project (3GPP) network, such as as a Long Term Evolution (LTE) radio access network (RAN) (e.g., over one or more LE bands), a New Radio (NR) RAN (e.g., over one or more NR bands), or a combination thereof. In some cases, the transceiver(s) 840 includes other wireless modems, such as a modem for engaging in WI-FI^{®}, WIGIG^{®}, WIMAX^{®}, BLUETOOTH^{®}, or infrared communication over the communication network(s) 842.

The defibrillator 800 is configured to transmit and/or receive data (e.g., ECG data, impedance data, data indicative of one or more detected heart rhythms of the individual 808, data indicative of one or more defibrillation shocks administered to the individual 808, etc.) with one or more external devices 844 via the communication network(s) 842. The external devices 844 include, for instance, mobile devices (e.g., mobile phones, smart watches, smart glasses, etc.), Internet of Things (loT) devices, medical devices, computers (e.g., laptop devices, servers, etc.), or any other type of computing device configured to communicate over the communication network(s) 842. In some examples, the external device(s) 844 is located remotely from the defibrillator 800, such as at a remote clinical environment (e.g., a hospital). According to various implementations, the processor(s) 812 causes the transceiver(s) 840 to transmit data to the external device(s) 844. In some cases, the transceiver(s) 840 receives data from the external device(s) 844 and the transceiver(s) 840 provide the received data to the processor(s) 812 for further analysis.

In various implementations, the external defibrillator 800 also includes a housing 846 that at least partially encloses other elements of the external defibrillator 800. For example, the housing 846 encloses the detection circuit 810, the processor(s) 812, the memory 814, the charging circuit 827, the transceiver(s) 840, or any combination thereof. In some cases, the input device(s) 818 and output device(s) 820 extend from an interior space at least partially surrounded by the housing 846 through a wall of the housing 846. In various examples, the housing 846 acts as a barrier to moisture, electrical interference, and/or dust, thereby protecting various components in the external defibrillator 800 from damage.

In some implementations, the external defibrillator 800 is an automated external defibrillator (AED) operated by an untrained user (e.g., a bystander, layperson, etc.) and can be operated in an automatic mode. In automatic mode, the processor(s) 812 automatically identifies a rhythm in the ECG signal, makes a decision whether to administer a defibrillation shock, charges the capacitor(s) 830, discharges the capacitor(s) 830, or any combination thereof. In some cases, the processor(s) 812 controls the output device(s) 820 to output (e.g., display) a simplified user interface to the untrained user. For example, the processor(s) 812 refrains from causing the output device(s) 820 to display a waveform of the ECG signal and/or the impedance signal to the untrained user, in order to simplify operation of the external defibrillator 800.

In some examples, the external defibrillator 800 is a monitor-defibrillator utilized by a trained user (e.g., a clinician, an emergency responder, etc.) and can be operated in a manual mode or the automatic mode. When the external defibrillator 800 operates in manual mode, the processor(s) 812 cause the output device(s) 820 to display a variety of information that may be relevant to the trained user, such as waveforms indicating the ECG data and/or impedance data, notifications about detected heart rhythms, and the like.

### EXAMPLE CLAUSES

1. A method, including: identifying an airway parameter indicative of breath events including a positive pressure ventilation (PPV) breath event; identifying, based on the airway parameter, a spontaneous breath event among the breath events; and performing an action based on the spontaneous breath event.
2. The method of clause 1, wherein the airway parameter includes a partial pressure of CO₂ in an airway, a flow rate of air in the airway, or a pressure of the air in the airway.
3. The method of clause 1 or 2, wherein the PPV breath event is administered by a ventilator or a bag-valve mask.
4. The method of any one of clauses 1 to 3, wherein the action includes outputting an indication of the spontaneous breath event, a warning that a patient is regaining consciousness, an instruction to administer anesthesia to the patient, or transmitting data indicating the spontaneous breath event to an external device.
5. The method of any one of clauses 1 to 4, further including: identifying, based on the airway parameter, the PPV breath event.
6. The method of clause 5, wherein performing the action based on the spontaneous breath event includes: generating data indicating the spontaneous breath event and the PPV breath event; and storing or transmitting the data.
7. A medical device including: a processor; and memory storing instructions that, when executed by the processor, cause the processor to perform operations including the method of any one of clauses 1 to 6.
8. The medical device of clause 7, further including: a transceiver configured to receive a signal indicative of the airway parameter.
9. The medical device of clause 7 or 8, further including: a sensor configured to detect the airway parameter.
10. The medical device of clause 9, wherein the sensor includes a bidirectional airflow sensor.
11. A method, including: identifying an airway parameter indicative of breath events including positive pressure ventilation (PPV) breath events; identifying, based on the airway parameter, spontaneous breath events among the breath events; and performing an action based on the spontaneous breath events.
12. The method of clause 11, further including: determining a rate of the spontaneous breath events, wherein performing the action based on the spontaneous breath events is further based on the rate of the spontaneous breath events.
13. The method of clause 11 or 12, the airway parameter being a first airway parameter, the method further including: determining a second airway parameter associated with the spontaneous breath events, wherein performing the action based on the spontaneous breath events is further based on the second airway parameter associated with the spontaneous breath events.
14. The method of clause 13, wherein the first airway parameter includes a partial pressure of CO₂ in an airway of a patient, and wherein the second airway parameter includes an end tidal CO₂ (EtCO₂) or a tidal volume.
15. The method of any one of clauses 11 to 14, further including: identifying, based on the airway parameter, the PPV breath events.
16. The method of clause 15, the action being a first action, the method further including: determining a rate of the PPV breath events; and performing a second action based on the rate of the PPV breath events.
17. The method of clause 15, the airway parameter being a first airway parameter, the action being a first action, the method further including: determining a second airway parameter associated with the PPV breath events; and performing a second action based on the second airway parameter.
18. A medical device including: a processor; and memory storing instructions that, when executed by the processor, cause the processor to perform operations including the method of any one of clauses 11 to 17.
19. The medical device of clause 18, further including: a transceiver configured to receive a signal indicative of the airway parameter.
20. The medical device of clause 18 or 19, further including: a sensor configured to detect the airway parameter.
21. The medical device of clause 20, wherein the sensor includes a bidirectional airflow sensor.
22. A method, including: identifying a partial pressure of CO₂ of an airway over time; identifying a breath event in the partial pressure of CO₂ of the airway over time; determining that the breath event lacks a plateau phase; determining an end-tidal CO₂ based on a maximum partial pressure of CO₂ in the breath event; outputting the end-tidal CO₂; and performing an action based on determining that the breath event lacks the plateau phase.
23. The method of clause 22, wherein determining that the breath event lacks the plateau phase includes: comparing a spectral signature of the breath event to a spectral signature of a standard breath event.
24. The method of clause 22 or 23, wherein determining that the breath event lacks the plateau phase includes: determining that a slope of the breath event following the maximum partial pressure of CO₂ in the breath event is greater than a threshold.
25. The method of any one of clauses 22 to 24, wherein determining that the breath event lacks the plateau phase includes: determining that a time interval between the maximum partial pressure of CO₂ and a local minimum partial pressure of CO₂ after the maximum partial pressure of CO₂ is greater than a threshold.
26. The method of any one of clauses 22 to 25, wherein the action includes outputting an indication that the end-tidal CO₂ is erroneously low or inaccurate.
27. The method of any one of clauses 22 to 26, wherein the action includes outputting an instruction to check for a leak between a ventilation device and an airway of a patient.
28. The method of any one of clauses 22 to 27, wherein the action includes outputting an instruction to use two hands to hold down a mask of a bag-valve mask disposed on a face of a patient.
29. A medical device including: a processor; and memory storing instructions that, when executed by the processor, cause the processor to perform operations including the method of any one of clauses 22 to 28.
30. The medical device of clause 29, further including: a transceiver configured to transmit a signal indicating that the breath event lacks the plateau phase.
31. The medical device of clause 29 or 30, further including: a sensor configured to detect the partial pressure of CO₂.
32. The method of any one of clauses 22 to 28, further including: identifying a flow rate through an airway of an individual; identifying a tidal volume of the individual based on the flow rate; and outputting the tidal volume of the individual with an indication that the tidal volume is erroneously low or inaccurate.
33. A medical device including: a processor; and memory storing instructions that, when executed by the processor, cause the processor to perform operations including the method of any one of clauses 22 to 28 or 32.
34. The medical device of clause 33, further including: a first sensor configured to detect the partial pressure of CO₂; and a second sensor configured to detect the flow rate, wherein the first sensor and the second sensor are disposed in a fluidic circuit including the airway of the individual.
35. A method, including: identifying a pressure in an airway of a patient over a time interval, wherein assisted ventilation is administered to the patient during the time interval; determining that the pressure exceeds a threshold pressure during the time interval; and performing an action based on the pressure exceeding the threshold pressure.
36. The method of clause 35, wherein the assisted ventilation is administered to the patient by a ventilation device manually operated by a rescuer.
37. The method of clause 36, wherein the ventilation device includes a bag-valve mask disposed on a face of the patient.
38. The method of clause 36 or 37, wherein the ventilation device includes an endotracheal tube or a supraglottic airway device disposed in an airway of the patient.
39. The method of any one of clauses 35 to 38, wherein determining that the pressure exceeds the threshold pressure during the time interval includes: determining a metric based on a fraction of the time interval during which the pressure exceeds the threshold pressure, and wherein performing the action includes outputting the metric, outputting an instruction based on the metric, outputting a warning based on the metric, transmitting data indicating the metric to an external device, causing the ventilation device to vent the airway of the patient, or causing the ventilation device to lower a ventilation rate or a positive pressure administered to the airway of the patient.
40. The method of clause 39, wherein the instruction includes an instruction to reduce the ventilation rate of the patient or an instruction to reduce a positive pressure administered to an airway of the patient.
41. The method of any one of clauses 35 to 40, wherein the threshold pressure is less than a pressure sufficient to open an esophageal sphincter of the patient or is less than a pressure sufficient to inhibit circulation of blood in lungs of the patient.
42. The method of any one of clauses 35 to 41, wherein performing the action includes outputting an indication of the pressure exceeding the threshold pressure, outputting a warning based on the pressure exceeding the threshold pressure, outputting an instruction based on the pressure exceeding the threshold pressure, transmitting an indication of the pressure exceeding the threshold pressure to an external device, causing the ventilation device to vent the airway of the patient, or causing the ventilation device to lower a ventilation rate or a positive pressure administered to the airway of the patient.
43. A medical device including: a processor; and memory storing instructions that, when executed by the processor, cause the processor to perform operations including the method of any one of clauses 35 to 42.
44. The medical device of clause 43, further including: a transceiver configured to transmit a signal indicating that the pressure exceeds the threshold pressure.
45. The medical device of clause 43 or 44, further including: a sensor configured to detect the pressure in the airway of the patient.
46. A method, including: identifying an airway parameter of an individual receiving assisted ventilation; determining a metric based on the airway parameter; and performing an action based on the metric.
47. The method of clause 46, wherein the airway parameter includes an airway pressure of the individual detected during a time interval, and determining the metric includes determining a fraction of the time interval at which the airway pressure exceeded a threshold.
48. The method of clause 46 or 47, wherein the airway parameter includes a CO₂ level in the airway of the individual, and wherein determining the metric includes determining, based on the CO₂ level, a number or rate of spontaneous breaths of the individual.
49. The method of any one of clauses 46 to 48, wherein the airway parameter includes a flow rate through an airway of the individual, and wherein determining the metric includes: determining, based on the flow rate, a tidal volume of the individual; and determining a variance of the tidal volume of the individual.
50. The method of any one of clauses 46 to 49, wherein performing the action includes outputting the metric, storing the metric, or transmitting data indicative of the metric to an external device.
51. The method of any one of clauses 46 to 50, wherein performing the action includes alarming based on the metric.
52. The method of clause 51, wherein alarming based on the metric includes determining that the metric is greater than a first threshold or less than a second threshold.
53. The method of clause 51 or 52, wherein alarming based on the metric includes outputting a warning, outputting an instruction to change a parameter of the assisted ventilation, outputting an instruction to check for a problem with a ventilation device providing the assisted ventilation, or outputting an instruction to administer anesthesia to the individual.
54. A medical device including: a processor; and memory storing instructions that, when executed by the processor, cause the processor to perform operations including the method of any one of clauses 46 to 53.
55. The medical device of clause 54, further including: a transceiver configured to receive a signal indicative of the airway parameter.
56. The medical device of clause 54 or 55, further including: a sensor configured to detect the airway parameter.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11 % of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1 % of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventor expects skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. A method, comprising:
identifying data indicating an airway parameter of an individual detected during a time interval, the data being indicative of breath events comprising a positive pressure ventilation (PPV) breath event;
identifying a spontaneous breath event among the breath events; and
in response to identifying the spontaneous breath event, outputting an indication of the spontaneous breath event.

2. The method of claim 1, wherein the airway parameter comprises a pressure of air in an airway of the individual, a flow rate of the air in the airway, a volume of the air in the airway, a rate of respiration and/or ventilation, an inspiratory time, an expiratory time, a partial pressure of CO₂ in the airway, a partial pressure of O₂ in the airway, an end tidal CO₂, or an end tidal O₂.

3. The method of claim 1 or 2, the spontaneous breath event being a first spontaneous breath, the data being first data, the time interval being a first time interval, the method further comprising:
identifying second data indicating the airway parameter of the individual detected during a second time interval;
determining that a medication has prevented the individual from breathing by determining that a portion of the second data lacks a second spontaneous breath event during the second time interval;
in response to determining that the medication has prevented the individual from spontaneously breathing, outputting a recommendation to connect a ventilation device to an airway of the individual.

4. The method of any of claims 1-3, wherein identifying the spontaneous breath event among the breath events comprising:
determining a spontaneous breathing index by analyzing the data, the spontaneous breathing index indicating a contribution of spontaneous breaths in breathing of the individual during the time interval; and
comparing the spontaneous breathing index to a threshold.

5. The method of any of claims 1-4, wherein identifying the spontaneous breath event among the breath events comprises:
determining a variability index by analyzing the data, the variability index indicating a variance of the airway parameter during the time interval; and
comparing the variability index to a threshold.

6. The method of any of claims 1-5, the airway parameter being a first airway parameter, the method further comprising:
detecting a second airway parameter of the individual during the time interval;
generating a corrected airway parameter by decreasing the second airway parameter; and
outputting the corrected airway parameter.

7. The method of any of claims 1-6, wherein outputting the indication of the spontaneous breath event comprises outputting an alert indicating that the individual is gaining spontaneous respiration, outputting an instruction to administer a medication to the individual, and/or transmitting data indicating the spontaneous breath event to an external device.

8. The method of any of claims 1-7, the data being first data, the time interval being a first time interval, the method further comprising:
identifying an input signal indicating that a ventilation device was placed during a second time interval;
identifying second data indicating the airway parameter of the individual detected during the second time interval;
determining that the second data indicates an absence of breath events during the second time interval; and
in response to determining that the second data indicates the absence of the breath events during the second time interval, outputting an alert indicating that the input signal is erroneous.

9. The method of any of claims 1-8, the data being first data, the time interval being a first time interval, the method further comprising:
identifying second data indicating the airway parameter of the individual detected during a second time interval;
determining that a leak is present between the ventilation device and the airway of the individual by analyzing the second data; and
in response to determining that the leak is present between the ventilation device and the airway of the individual ouputtting an alert indicating the leak.

10. A medical device, comprising:
a sensor configured to detect an airway parameter of an individual receiving positive pressure ventilation (PPV) from a ventilation device;
an output device; and
a processor configured to:
determine that a leak is present between the ventilation device and an airway of the individual by analyzing the airway parameter; and
in response to determining that the leak is present between the ventilation device and the airway of the individual, cause the output device to output an alert indicating the leak.

11. The medical device of claim 10, wherein the airway parameter comprises a pressure of air in the airway, a flow rate of the air in the airway, a volume of the air in the airway, a rate of respiration and/or ventilation, an inspiratory time, an expiratory time, a partial pressure of CO₂ in the airway, a partial pressure of O₂ in the airway, an/or end tidal CO₂, or an end tidal O₂.

12. The medical device of claim 11, wherein the airway parameter comprises a partial pressure of CO₂ in the airway of the individual, and
wherein the processor is configured to determine that the leak is present between the ventilation device and the airway of the individual by determining that the airway parameter lacks a plateau phase.

13. The medical device of claim 11 or 12, wherein the airway parameter comprises a partial pressure of O₂ in the airway of the individual, and
wherein the processor is configured to determine that the leak is present between the ventilation device and the airway of the individual by determining that the airway parameter is below a threshold.

14. The medical device of claim 11, 12 or 13, wherein the airway parameter comprises a volume of air in the airway of the individual, and
wherein the processor is configured to determine that the leak is present between the ventilation device and the airway of the individual by determining that the airway parameter is lower than a volume of air output by the ventilation device.

15. The medical device of any of claims 10-14, wherein the processor is configured to determine that the leak is present between the ventilation device and the airway of the individual by analyzing first data indicating the airway parameter detected by the sensor during a first time interval, and
wherein the processor is further configured to:
determine that a medication has prevented the individual from breathing by analyzing second data indicating the airway parameter detected by the sensor during a second time interval; and
based on determining that the medication has prevented the individual from spontaneously breathing, cause the output device to output a recommendation to connect the ventilation device to the airway of the individual.

16. The medical device of any of claims 10-15, wherein the processor is configured to determine that the leak is present between the ventilation device and the airway of the individual by analyzing first data indicating the airway parameter detected by the sensor during a first time interval, and
wherein the processor is further configured to:
identify breath events by analyzing second data indicating the airway parameter detected by the sensor during a second time interval, the breath events comprising a positive pressure ventilation (PPV) breath event;
identify a spontaneous breath event among the breath events; and
in response to identifying the spontaneous breath event, outputting an indication of the spontaneous breath event.

17. The medical device of any of claims 10-16, further comprising:
a blood pressure sensor configured to detect a blood pressure of the individual,
wherein the airway parameter comprises an end tidal CO₂, and
wherein the processor is further configured to:
determine a time interval during which the airway parameter is less than a first threshold and a respiration rate of the individual is greater than a second threshold;
determine that the individual is being hyperventilated by determining a hyperventilation index using the time interval; and
in response to determining that the individual is being hyperventilated, cause the blood pressure sensor to detect the blood pressure of the individual.

18. A system, comprising:
a ventilation device configured to be connected to an airway of a patient;
a CO₂ sensor configured to detect a partial pressure of CO₂ in the airway of the patient;
a display configured to output a first waveform indicating the partial pressure of CO₂ during a time interval and a second waveform indicating an airway parameter that is different than the partial pressure of CO₂ during the time interval; and
a processor configured to:
identify a breath event in the partial pressure of CO₂ of the airway during the time interval;
identify a leak between the ventilation device and the airway by determining that the breath event lacks a plateau phase by:
determining that a slope of the breath event following the maximum partial pressure of CO₂ in the breath event is greater than a first threshold; or
determining that a time interval between a maximum partial pressure of CO₂ and a local minimum partial pressure of CO₂ after the maximum partial pressure of CO₂ is greater than a second threshold; and
in response to determining that the breath event lacks the plateau phase:
cause the display to output a first alert warning to check for the leak between the ventilation device and the airway of the patient; and
cause the display to output a second alert indicating that the airway parameter indicated by the second waveform is erroneous.

19. The system of claim 18, the time interval being a first time interval, wherein the processor is further configured to:
determine that a paralytic has prevented the individual from breathing by analyzing first data, the first data indicating the airway parameter during a second time interval;
based on determining that the paralytic has prevented the individual from spontaneously breathing, cause the display to output a recommendation to connect the ventilation device to the airway of the individual;
identify breath events by analyzing second data, the second data indicating the airway parameter during a third time interval, the breath events comprising a positive pressure ventilation (PPV) breath event;
identify a spontaneous breath among the breath events; and
in response to identifying the spontaneous breath event, cause the display to output an indication of the spontaneous breath event;

20. The system of claim 18 or 19, the time interval being a first time interval, the system further comprising:
a blood pressure sensor configured to detect a blood pressure of the individual,
wherein processor is further configured to:
determine a second time interval during which the partial pressure of CO₂ is less than a first threshold and a respiration rate of the individual is greater than a second threshold;
determine that the individual is being hyperventilated by determining a hyperventilation index proportional to a duration of the second time interval; and
in response to determining that the individual is being hyperventilated, cause the blood pressure sensor to detect the blood pressure of the individual.
